# EUROPEAN PATENT APPLICATION

(11) **EP 3 998 086 A1**
(43) Date of publication of application: **18.05.2022**
(21) Application number: 20840269.3
(22) Date of filing: 10.07.2020
(51) Int. Cl.: A61K 48/00, A61P 27/02, A61P 27/06, C12N 15/12, C12N 15/55, C12N 15/63, A61K 38/46, A61K 31/7088, A61K 31/7105, A61K 31/711

(54) **THERAPEUTIC AGENT FOR DISEASE CAUSED BY DOMINANT MUTANT GENE**

(30) Priority: 12.07.2019 JP 2019130199
(71) Applicant: RIKEN, Wako-shi Saitama 351-0198 (JP)
(72) Inventor: TAKAHASHI, Masayo, Wako-shi, Saitama 351-0198 (JP); ONISHI, Akishi, Wako-shi, Saitama 351-0198 (JP); TSUNEKAWA, Yuji, Wako-shi, Saitama 351-0198 (JP)
(74) Representative: Graf von Stosch Patentanwaltsgesellschaft mbH
(86) International application number: PCT/JP2020/026956
(87) International publication number: WO 2021/010303

(57) **Abstract**

Provided is a novel therapeutic agent for a disease caused by a dominant gene mutation (7). A therapeutic agent of the present invention comprises a donor DNA (20) that contains a polynucleotide having the following sequences (a) to (c): (a) a normal gene (1); (b) a first reverse target DNA (2a) that is located upstream of the normal gene (1) and that is cleaved by a designer nuclease; and (c) a second reverse target DNA (2b) that is located downstream of the normal gene (1) and that is cleaved by the designer nuclease, where the reverse target DNAs (2a, 2b) each mean a sequence obtained by inverting a target sequence (6) that is present in the genome and that is cleaved by the designer nuclease.

## Description

### Technical Field

The present invention relates to a therapeutic agent for a disease caused by a dominant gene mutation.

### Background Art

Diseases caused by a gene mutation include a disease caused by a recessive gene mutation and a disease caused by a dominant gene mutation. The recessive diseases occur when the mutation is homozygous, whereas the dominant diseases occur even when the mutation is heterozygous. This is because the proteins with recessive mutations do not inhibit the function of the normal proteins expressed from the normal allele, whereas the proteins with dominant mutations inhibit the function of the normal proteins expressed from the normal allele and/or acquire an activity greater than or equal to that of normal protein so as to cause excessive cellular response. Other examples of a dominant mutation include a case where a mutation results in a lack of the normal proteins expressed from the normal allele (haploinsufficiency).

Due to such differences, the gene therapy strategies are different between the disease caused by recessive gene mutations and the disease caused by dominant gene mutations. Regarding the recessive diseases, a therapeutic method of introducing a normal gene into a patient is being developed. Regarding the dominant diseases, a therapeutic method is necessary to be developed in which the mutated nucleotide sequence in a genome is exchanged for the normal sequence, and such a therapeutic method is currently required to be developed.

In the field of basic studies, development of genome editing technologies has recently been attracting attention, and various genome editing technologies are being developed (see Patent Literatures 1 and 2). Patent Literature 1 discloses a method of bringing a target nucleic acid into contact with Cas9 polypeptide and DNA-targeting RNA so as to regulate transcription of the target nucleic acid. Patent Literature 2 discloses a method of incorporating an exogenous DNA sequence into a genome of a non-dividing cell without relying on homology. There is also an attempt to apply such genome editing technologies to various fields.

### Citation List

### [Patent Literatures]

[Patent Literature 1]
   Pamphlet of International Publication No. WO 2013/176772
[Patent Literature 2]
   Pamphlet of International Publication No. WO 2018/013932

### Summary of Invention

### Technical Problem

It is determined by pedigree analysis and gene diagnosis whether a gene mutation is recessive or dominant. However, previous diagnostic knowledge makes it possible to distinguish between a recessive gene mutation and a dominant gene mutation in accordance with disease causative genes (e.g., a rhodopsin gene has a dominant mutation, and the Usherin gene has a recessive mutation). The site of mutation often differs among patients, even if the disease is caused by the same causative gene. Therefore, in treatment of a disease caused by a dominant gene mutation, a mutated nucleotide needs to be exchanged for a normal nucleotide depending on each patient. However, a problem that such an operation requires a great deal of labor and cost hinders the drug formulation development.

The present invention has been made in view of the problems described above, and an object of the present invention is to provide a novel therapeutic agent for a disease caused by a dominant gene mutation.

### Solution to Problem

To solve the issue as written above, a therapeutic agent in accordance with an aspect of the present invention is
a therapeutic agent for a disease caused by a dominant gene mutation in which a dominant mutation occurs in a normal gene in a genome,
the therapeutic agent comprising a donor DNA that contains a polynucleotide having the following sequences (a) to (c):
   (a) the normal gene;
   (b) a first reverse target DNA that is located upstream of the normal gene and that is cleaved by a designer nuclease; and
   (c) a second reverse target DNA that is located downstream of the normal gene and that is cleaved by the designer nuclease,
where the first reverse target DNA and the second reverse target DNA each mean a sequence obtained by inverting a target sequence that is present in the genome and that is cleaved by the designer nuclease.

### Advantageous Effects of Invention

An aspect of the present invention provides a novel therapeutic agent for a disease caused by a dominant gene mutation.

### Brief Description of Drawings

Fig. 1 is a view schematically illustrating a function of a therapeutic agent in accordance with an aspect of the present invention.
Fig. 2 is a view schematically illustrating an example in which a therapeutic agent in accordance with an embodiment of the present invention is applied to repair of a rhodopsin gene.
Fig. 3 is a view illustrating a defect that appears during application of a conventional genome editing technology to repair of a rhodopsin gene.
Fig. 4 is a view illustrating three types of gRNA studied in a rhodopsin gene repair experiment.
Fig. 5 is a view schematically illustrating a structure of a donor DNA used in the rhodopsin gene repair experiment.
Fig. 6 is a view schematically illustrating a method for carrying out the rhodopsin gene repair experiment.
Fig. 7 is a view showing a microscopic image that shows a result of the rhodopsin gene repair experiment. The view illustrates rhodopsin expression in the retina.
Fig. 8 is a view showing a microscopic image that shows a result of the rhodopsin gene repair experiment. The view illustrates rhodopsin expression in a section of the retina.
Fig. 9 is a graph showing a result of the rhodopsin gene repair experiment. The graph shows knock-in efficiency for each gRNA.
Fig. 10 is a view showing a microscopic image that shows a result of the rhodopsin gene repair experiment. The view shows a fluorescence stained image in which an anti-rhodopsin antibody is used.
Fig. 11 is a view showing a microscopic image that shows a result of the rhodopsin gene repair experiment by non-viral delivery (electroporation). The view illustrates rhodopsin expression in the retina.
Fig. 12 is a view showing a microscopic image that shows a result of the rhodopsin gene repair experiment by viral delivery (an AAV vector). The view illustrates rhodopsin expression in the retina.
Fig. 13 is a view showing a microscopic image that shows a result of the rhodopsin gene repair experiment by viral delivery (an AAV vector). The view illustrates rhodopsin expression in a section of the retina.
Fig. 14 is a view showing a microscopic image that shows a result of the rhodopsin gene repair experiment. The view shows that knock-in of a normal rhodopsin gene suppresses degeneration of the retina.
Fig. 15 is a view showing a microscopic image that shows a result of the rhodopsin gene repair experiment. The view confirms rhodopsin expression in the retina by an eyeground image.
Fig. 16 is a graph showing a result of the rhodopsin gene repair experiment. The graph shows a result of an optokinetic response test (qOMR).
Fig. 17 is a view illustrating a position of a gRNA recognition sequence, which position is selected upstream of an exon 1 of a human rhodopsin gene.
Fig. 18 is a view schematically illustrating an SSA assay for verifying efficiency of cleavage of a gRNA recognition sequence.
Fig. 19 is a view showing a microscopic image that shows a result of the SSA assay. The view illustrates expression of EGFP.
Fig. 20 is a view schematically illustrating a structure of a donor DNA of a normal human rhodopsin gene.
Fig. 21 is a view illustrating positions of three types of gRNA recognition sequences studied in a peripherin gene repair experiment.
Fig. 22 is a view schematically illustrating a structure of a donor DNA used in the peripherin gene repair experiment.
Fig. 23 is a view showing a microscopic image that shows a result of the peripherin gene repair experiment. The view illustrates expression of peripherin.

### Description of Embodiments

The following description will discuss embodiments of the present invention. The present invention is not, however, limited to these embodiments. The present invention is not limited to any configurations described below, and can be altered in various ways within the scope of the claims. The present invention also encompasses, in its technical scope, any embodiment and/or Example derived by combining technical means disclosed in differing embodiments and/or Examples.

All academic and patent documents cited in the present specification are incorporated herein by reference.

Any numerical range expressed as "A to B" herein means "not less than A (A or more) and not more than B (B or less)" unless otherwise specified.

### [1. Therapeutic agent for disease caused by dominant gene mutation]

In an embodiment, the present invention provides a therapeutic agent for a disease caused by a dominant gene mutation in which a dominant mutation occurs in a normal gene in a genome. The therapeutic agent contains a donor DNA that contains a polynucleotide having the following sequences (a) to (c): (a) a normal gene; (b) a first reverse target DNA that is located upstream of the normal gene and that is cleaved by a designer nuclease; and (c) a second reverse target DNA that is located downstream of the normal gene and that is cleaved by the designer nuclease.

### [Action mechanism of therapeutic agent]

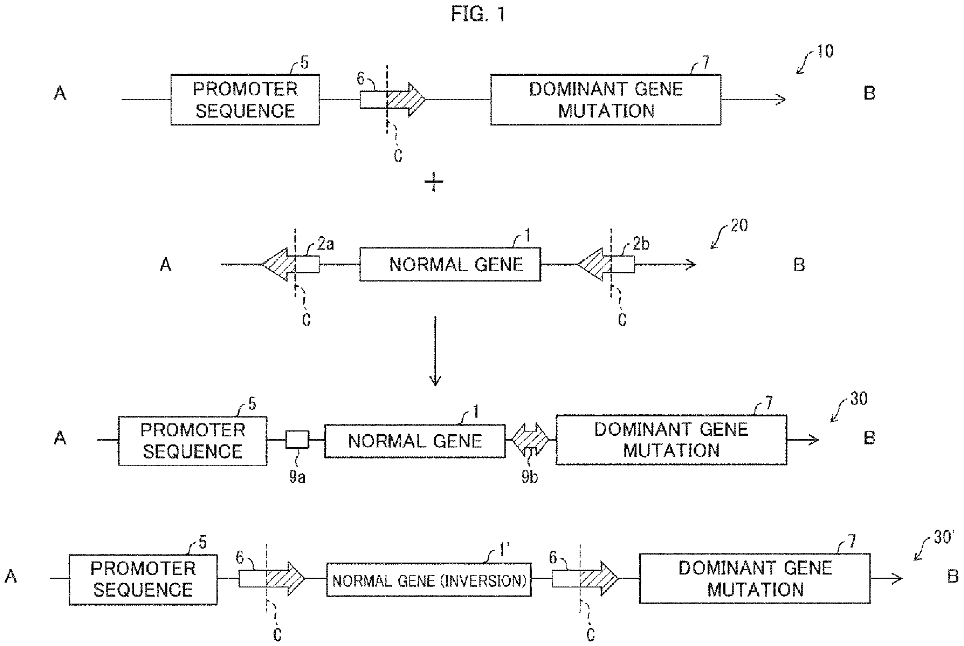

The following description will discuss, with reference to Fig. 1, an action mechanism of a therapeutic agent in accordance with an aspect of the present invention. Fig. 1 illustrates a genome 10 before treatment, a donor DNA 20, and a genome 30 after treatment. The therapeutic agent in accordance with an embodiment of the present invention contains the donor DNA 20. The donor DNA 20 includes a normal gene 1, and knock-in of the normal gene 1 into the genome causes a dominant gene mutation 7 to be knocked out. This results in treatment of a disease caused by the dominant gene mutation 7.

In Fig. 1, the genome 10 before treatment, the donor DNA 20, and the genome 30 after treatment are all illustrated in a direction from an upstream A toward a downstream B. In the genome 10 before treatment and the genome 30 after treatment, with respect to a nucleotide chain in which the dominant gene mutation 7 that is endogenous is encoded, a 5' side is "upstream", and a 3' side is "downstream". With respect to a nucleotide chain that is complementary to the above nucleotide chain, the 3' side is "upstream", and 5' side is "downstream".

Meanwhile, in the donor DNA 20, with respect to a nucleotide chain in which the normal gene 1 is encoded, the 5' side is "upstream", and the 3' side is "downstream". With respect to a nucleotide chain that is complementary to the above nucleotide chain, the 3' side is "upstream", and 5' side is "downstream". Note that the donor DNA 20 can be a single-stranded DNA having no complementary nucleotide chain.

The first row of Fig. 1 is a view schematically illustrating the genome 10 before treatment by the therapeutic agent in accordance with an embodiment of the present invention. The genome 10 before treatment includes a promoter sequence 5 and the dominant gene mutation 7. This results in a situation where a translation product of the dominant gene mutation 7 is produced, so that various diseases are caused.

A target sequence 6 is present between the promoter sequence 5 and the dominant gene mutation 7, and the target sequence 6 includes a cleavage site C. In an embodiment, at least a part of the target sequence 6 is recognized by a properly designed nucleic acid binding site of a designer nuclease, and the cleavage site C is cleaved by a nuclease site of the designer nuclease. Examples of the designer nuclease of this embodiment include a TALE nuclease (TALEN) and a zinc-finger nuclease (ZFN). In another embodiment, at least a part of the target sequence 6 is recognized by a properly designed gRNA, and the cleavage site C is cleaved by the nuclease site of the designer nuclease. Examples of the designer nuclease of this embodiment include a Cas nuclease. In this case, DNA that is cleaved by the designer nuclease preferably has a flush end. This is because DNA that has a flush cleavage end facilitates insertion of a normal gene by DNA repair by NHEJ after cleavage.

Examples of the designer nuclease that satisfies such conditions include a Cas nuclease (CRISPR-related nuclease; including a natural Cas nuclease (Cas9 etc.) and an artificial mutant (dCas etc.)), a TALE nuclease (TALEN), a zinc-finger nuclease (ZFN), and a pentatricopeptide repeat (PPR) protein.

A position of the target sequence 6 is not particularly limited and can be any position at which the normal gene 1 can be controlled by the promoter sequence 5 in response to insertion of the normal gene 1 into a location of the cleavage site C. The target sequence 6 can be positioned, for example, (i) between the promoter sequence 5 and the dominant gene mutation 7 or (ii) within the dominant gene mutation 7 (e.g., within the exon 1).

The second row of Fig. 1 is a view schematically illustrating the donor DNA 20 contained in the therapeutic agent in accordance with an embodiment of the present invention. The donor DNA 20 includes a polynucleotide having sequences, which are the normal gene 1, a first reverse target DNA 2a, and a second reverse target DNA 2b. The first reverse target DNA 2a, the normal gene 1, and the second reverse target DNA 2b are arranged in order from upstream.

The normal gene 1 means a gene encoding substantially normally functioning protein. Note here that protein that has a plurality of functions at least one of which is substantially normal can be regarded as "substantially normally functioning protein". For example, in a case where protein has a function that is a certain activity (gene expression activity etc.), an intensity of the activity as measured by an appropriate assay may be not less than 80%, not less than 90%, or not less than 95% of an activity of wild type protein (an upper limit of the intensity may be not more than 120%, not more than 110%, or not more than 105%). In an embodiment, the normal gene 1 is a wild type gene.

The sequences, which are the first reverse target DNA 2a and the second reverse target DNA 2b, are obtained by inverting the sequence of the target sequence 6. That is, a base sequence observed when the target sequence 6 is read from the upstream side matches base sequences observed when the first reverse target DNA 2a and the second reverse target DNA 2b are read from the downstream side. Thus, the first reverse target DNA 2a and the second reverse target DNA 2b each also include the cleavage site C.

The third row of Fig. 1 is a view schematically illustrating the genome 30 after treatment by the therapeutic agent in accordance with an embodiment of the present invention. Action of the designer nuclease (not illustrated) causes the normal gene 1 in the donor DNA to be inserted between the promoter sequence 5 and the dominant gene mutation 7 with use of non-homologous end repair (NHEJ). The dominant gene mutation 7 that is inserted downstream of the promoter sequence 5 causes the promoter sequence 5 to produce a product of the normal gene 1 (knock-in). Meanwhile, since the normal gene 1 has an end at which at least a stop codon is positioned, leakage (expression leakage) of the dominant gene mutation 7 which leakage is caused by transcription of the normal gene 1 does not occur.

Note that a gRNA (not illustrated) can be involved in the above mechanism. For example, in a case where the designer nuclease is a Cas nuclease, the Cas nuclease and the gRNA work together to cleave a double-stranded DNA chain.

Here, the target sequence 6, which was present in the genome 10 before treatment, has disappeared after treatment (9a, 9b). Thus, the normal gene 1 that is once inserted is not removed by the action of the designer nuclease.

In NHEJ, an insertion sequence may be inserted by inversion with respect to an intended direction (the fourth row of Fig. 1). The genome 30' that has been inserted by inversion includes a normal gene (inversion) 1'. Occurrence of such insertion prevents production of a normal product of the normal gene 1. However, in the genome 30' inserted by inversion, target sequences 6 are located upstream and downstream of the normal gene (inversion) 1'. Thus, the action of the designer nuclease removes the normal gene (inversion) 1', and NHEJ is used again to repair the genome.

The therapeutic agent in accordance with an embodiment of the present invention thus makes it possible to insert the normal gene 1 in a correct direction while using NHEJ.

### [Various aspects of therapeutic agent]

In an embodiment, the therapeutic agent includes a gRNA and/or a designer nuclease. The gRNA and/or the designer nuclease can be included in the therapeutic agent as a form of an expression vector, as a form of RNA, or as a form of protein. The gRNA and the designer nuclease can be fused together into a single structure.

The above aspect allows the therapeutic agent to include main elements required for genome editing. This makes it possible to use only the therapeutic agent to more efficiently treat a disease caused by a dominant gene mutation.

In an embodiment, the donor DNA 20 includes a transcriptional regulatory sequence between the normal gene 1 and the second reverse target DNA 2b. The transcriptional regulatory sequence is a sequence that regulates (e.g., enhances or suppresses) production of a transcription product (mRNA) from a gene. In an embodiment, the transcriptional regulatory sequence is a transcriptional inhibitory sequence. In an embodiment, the transcriptional regulatory sequence is an untranslated sequence (3'-side untranslated region) that is positioned on the 3' end side of a gene. Other specific examples of the transcriptional regulatory sequence include polyA addition sequences (SV40pA, rabbit globin polyA, and bGH polyA). It is also possible to employ, as the transcriptional regulatory sequence, a sequence obtained by combining a polyA addition sequence and an insulator sequence (boundary sequence; a sequence to prevent an influence caused by a sequence outside an inserted gene).

The above aspect allows the transcriptional regulatory sequence to be located between the normal gene 1 and the dominant gene mutation 7 in the genome 30 after treatment. This makes it possible to, for example, more reliably inhibit leakage (expression leakage) of the dominant gene mutation 7 which leakage is caused by transcription of the normal gene 1. Such an aspect is of particularly advantageous in a cell in which the dominant gene mutation 7 is strongly expressed.

In an embodiment, the therapeutic agent is used for a non-dividing cell. As described above, NHEJ is used in the therapeutic agent in accordance with an embodiment of the present invention. The therapeutic agent is therefore preferably applied to a cell in which DNA chain cleavage is repaired by NHEJ with high efficiency. Note here that NHEJ is commonly more frequently used in a non-dividing cell than in a dividing cell to repair DNA chain cleavage. The therapeutic agent is therefore preferably used for a non-dividing cell. Examples of the non-dividing cell include photoreceptor cells (a rod photoreceptor cell and a cone photoreceptor cell), a retinal pigment epithelial cell, and an optic nerve cell.

In an embodiment, the normal gene 1 is at least one gene selected from the group consisting of a rhodopsin gene, a peripherin gene, a BEST1 gene, and an OPTN gene. In an embodiment, the normal gene 1 is a rhodopsin gene, a peripherin gene, a BEST1 gene, or an OPTN gene.

In an embodiment, the disease caused by a dominant gene mutation is at least one disease selected from the group consisting of retinitis pigmentosa, macular dystrophy, and hereditary glaucoma. In an embodiment, the disease caused by a dominant gene mutation is retinitis pigmentosa, macular dystrophy, or hereditary glaucoma.

The normal gene 1 can have any structure that is not particularly limited, provided that a normal gene product can be obtained. The normal gene 1 can have therein an untranslated region, or can have no untranslated region. In an embodiment, the normal gene 1 is a cDNA of a normal gene product. In an embodiment, the normal gene 1 is a cDNA of a wild-type gene product.

The normal gene 1 that has no untranslated region allows the donor DNA 20 to have a smaller genome size. The normal gene 1 that has no untranslated region is therefore advantageous in a case where the donor DNA 20 is incorporated into a vector (a plasmid vector, a viral vector, etc.).

A vector that is used to introduce, into a cell, components contained in the therapeutic agent is not limited to any particular vector. Examples of the components of the therapeutic agent which components may be introduced by the vector include the donor DNA 20, an expression vector for the gRNA, and an expression vector for the designer nuclease. A known technique can be used to use various vectors to introduce the components into a cell.

It is also possible to configure, as a single vector, at least two structures selected from the group consisting of the donor DNA 20, the gRNA, and the designer nuclease. For example, it is possible to configure, as a single vector, (i) the gRNA and the designer nuclease, (ii) the donor DNA 20 and the gRNA, (iii) the donor DNA 20 and the designer nuclease, or (iv) the donor DNA 20, the gRNA, and the designer nuclease.

Specific examples of the vector include phage vectors, plasmid vectors, viral vectors, retroviral vectors, chromosomal vectors, episomal vectors, virus-derived vectors (bacterial plasmids, bacteriophages, yeast episomes, etc.), yeast chromosomal elements, viruses (baculoviruses, papovaviruses, vaccinia viruses, adenoviruses, adeno-associated viruses, avian pox viruses, pseudorabies viruses, herpes viruses, lentiviruses, retroviruses, etc.), and vectors derived from combinations thereof (cosmids, phagemids, etc.). Among the vectors listed above, a plasmid vector is preferable due to its high versatility. From the viewpoint of progress in clinical application, a viral vector is preferable, and an adeno-associated viral vector (AAV vector) is more preferable.

Alternatively, the components contained in the therapeutic agent can be introduced into a cell without use of any vector. For example, the donor DNA, the gRNA, and the designer nuclease can be introduced as a DNA molecule, an RNA molecule, and protein, respectively, into a cell. Examples of such an introduction method include electroporation, microinjection, sonoporation, laser irradiation, and transfection with use of combination with a cationic substance (a cationic polymer, a cationic lipid, calcium phosphate, etc.).

### [Kit]

An aspect of the present invention is a kit for treating a disease caused by a dominant gene mutation in which a dominant mutation occurs in a normal gene in a genome. This kit includes the donor DNA (described above).

In a case where the kit includes the gRNA (or an expression vector therefor) and/or the designer nuclease (or an expression vector therefor), the donor DNA, the gRNA, and the designer nuclease can be formulated as a single reagent or can be formulated separately as two or more reagents (for example, (a) the donor DNA and (b) the gRNA and the designer nuclease can be formulated as different reagents). In this case, the two or more reagents may be stored in different containers.

In an embodiment, the term "kit" means a combination of, for example, any reagents which combination is used in any application. The application can be a medical application or an experimental application.

### [Formulation, dosage form, and prescription]

The therapeutic agent in accordance with an embodiment of the present invention can be formulated by a usual method. More specifically, the therapeutic agent can be formulated by mixing the donor DNA (described above), optionally the gRNA (or an expression vector therefor) and/or the designer nuclease (or an expression vector therefor), and optionally a pharmaceutical additive.

The pharmaceutical additive herein means a substance different from an active ingredient contained in a preparation. The preparation contains the pharmaceutical additive so as to, for example, (i) be easily formulated, (ii) be more stable in quality, and (iii) be more useful. Examples of the pharmaceutical additive include excipients, binders, disintegrators, lubricants, fluidizing agents (solid inhibitors), coloring agents, capsule coatings, coating agents, plasticizers, flavoring agents, sweetening agents, aromatizing agents, solvents, solubilizers, emulsifying agents, suspending agents (adhesives), viscous agents, pH adjusting agents (acidifying agents, alkalizing agents, buffering agents), wetting agents (solubilizing agents), antimicrobial preservatives, chelating agents, suppository bases, ointment bases, curing agents, softening agents, medical water, propellants, stabilizers, and preservatives. These pharmaceutical additives are easily selected by a person skilled in the art in accordance with an intended dosage form and an intended administration route, and a standard pharmaceutical practice.

The therapeutic agent in accordance with an embodiment of the present invention can contain active ingredients different from the donor DNA, the gRNA, and the designer nuclease. The active ingredients can have an effect related to treatment of a disease caused by a dominant gene mutation, or can have other effect(s).

Specific examples of the active ingredient (described above) and the pharmaceutical additive (described above) can be found from standards developed by, for example, Food and Drug Administration (FDA), European Medicines Agency (EMA), and the Ministry of Health, Labour and Welfare of Japan.

The therapeutic agent in accordance with an embodiment of the present invention can take any dosage form. Examples of the dosage form include eye drops, tablets, capsules, internal preparations, external preparations, suppositories, injections, and inhalants.

The therapeutic agent in accordance with an embodiment of the present invention can be prescribed as appropriate at the discretion of physicians or health care professionals. A dose and a dosage regimen of the therapeutic agent in accordance with an embodiment of the present invention can also be determined as appropriate at the discretion of physicians or health care professionals.

An administration route of the therapeutic agent in accordance with an embodiment of the present invention is selected as appropriate in accordance with elements such as a dosage form of the therapeutic agent and a type and severity of a disease to be treated. Examples of the administration route include eye-drop administration, parenteral administration, intradermal administration, intramuscular administration, intraperitoneal administration, intravenous administration, subcutaneous administration, intranasal administration, epidural administration, oral administration, sublingual administration, intranasal administration, intracerebral administration, intravaginal administration, transcutaneous administration, intrarectal administration, inhalation, and local administration.

A "subject" to which the therapeutic agent in accordance with an embodiment of the present invention is to be administered is not limited to a human. The therapeutic agent can be applied to a non-human mammal other than a human. Examples of the non-human mammal include artiodactyls (cattle, wild boars, pigs, sheep, goats, etc.), perissodactyls (horses etc.), rodents (mice, rats, hamsters, squirrels, etc.), Lagomorpha (rabbits etc.), and carnivores (dogs, cats, ferrets, etc.). The non-human mammals listed above also encompass not only livestock or companion animals (pet animals) but also wild animals.

The therapeutic agent in accordance with an embodiment of the present invention can also be used not only for an organism. For example, the therapeutic agent can also be used for a system derived from an organism (an extracted tissue, a cultured cell, etc.).

### [2. Therapeutic agent for disease derived from rhodopsin gene in which dominant mutation occurs]

In an embodiment, the present invention provides a therapeutic agent for a disease caused by a dominant rhodopsin gene mutation in which a dominant mutation occurs in a normal rhodopsin gene in a genome. The therapeutic agent contains a donor DNA that contains a polynucleotide having the following sequences (a) to (c): (a) a normal rhodopsin gene; (b) a first reverse target DNA that is located upstream of the normal rhodopsin gene and that is cleaved by a designer nuclease; and (c) a second reverse target DNA that is located downstream of the normal rhodopsin gene and that is cleaved by the designer nuclease.

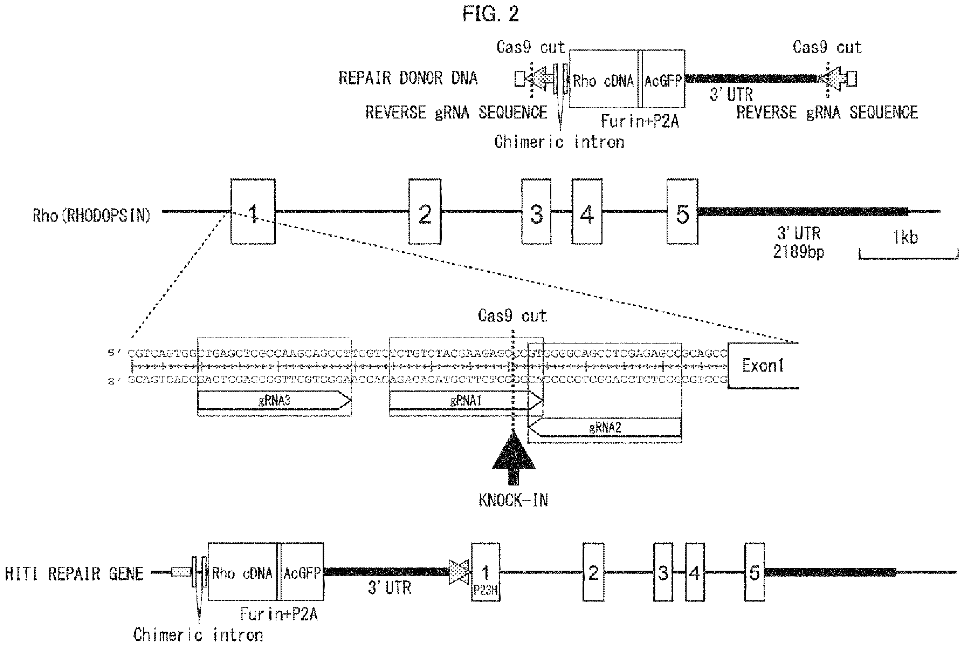

The first row of Fig. 2 illustrates an example of configuration of such a donor DNA. In Fig. 2, "Rho cDNA" is a cDNA of normal type rhodopsin protein and corresponds to the "normal rhodopsin gene". "REVERSE gRNA SEQUENCE" corresponds to each of the first reverse target DNA and the second reverse target DNA. In the example of Fig. 2, since Cas9 is used as the designer nuclease, the first reverse target DNA and the second reverse target DNA are each a sequence recognized by the gRNA. AcGFP is a marker protein that determines whether the normal rhodopsin gene has been successfully inserted, and does not need to be contained in the therapeutic agent.

The therapeutic agent knocks in the normal rhodopsin gene between a promoter of an endogenous rhodopsin gene and the exon 1 of the dominant rhodopsin gene mutation. As a result, the dominant rhodopsin gene mutation is knocked out (the middle row and the lower row of Fig. 2).

In an embodiment, the therapeutic agent contains a gRNA and/or a designer nuclease. The gRNA and/or the designer nuclease can be contained, as a form of an expression vector, in the therapeutic agent. The gRNA and the designer nuclease can be fused together into a single structure. In an embodiment, the donor DNA includes a transcriptional regulatory sequence between the normal rhodopsin gene and the second reverse target DNA ("3'UTR" corresponds to the transcriptional regulatory sequence in Fig. 2). Since these points are as described in section [1], a further description thereof will be omitted.

In an embodiment, the donor DNA further includes a sequence that causes the normal rhodopsin gene to be highly expressed. In Fig. 2, "Chimeric intron" corresponds to the sequence. This sequence is a chimeric intron derived from a human β globin gene and an immunoglobulin gene, and is known to increase the expression amount of protein in a cultured cell system (Choi T et al. (1991) "A Generic Intron Increases Gene Expression in Transgenic Mice," Molecular and Cellular Biology, Vol. 11 (No. 6), pp. 3070-3074; Sakurai K et al. (2007) "Physiological Properties of Rod Photoreceptor Cells in Green-sensitive Cone Pigment Knock-in Mice," Journal of General Physiology, Vol. 130 (No.1), pp. 21-40.).

In an embodiment, an expression cassette of the normal rhodopsin gene is inserted in a pLeaklessIII plasmid. That is, the donor DNA is inserted in the pLeaklessIII plasmid. The pLeaklessIII plasmid is a vector in which an influence of an insert on a transcriptional activity due to an endogenous cause of a plasmid has been reduced (for details see Tunekawa Y et al. (2016) "Developing a de novo targeted knock-in method based on in utero electroporation into the mammalian brain," Deveopment, Vol. 143 (Issue 17), pp. 3216-3222.).

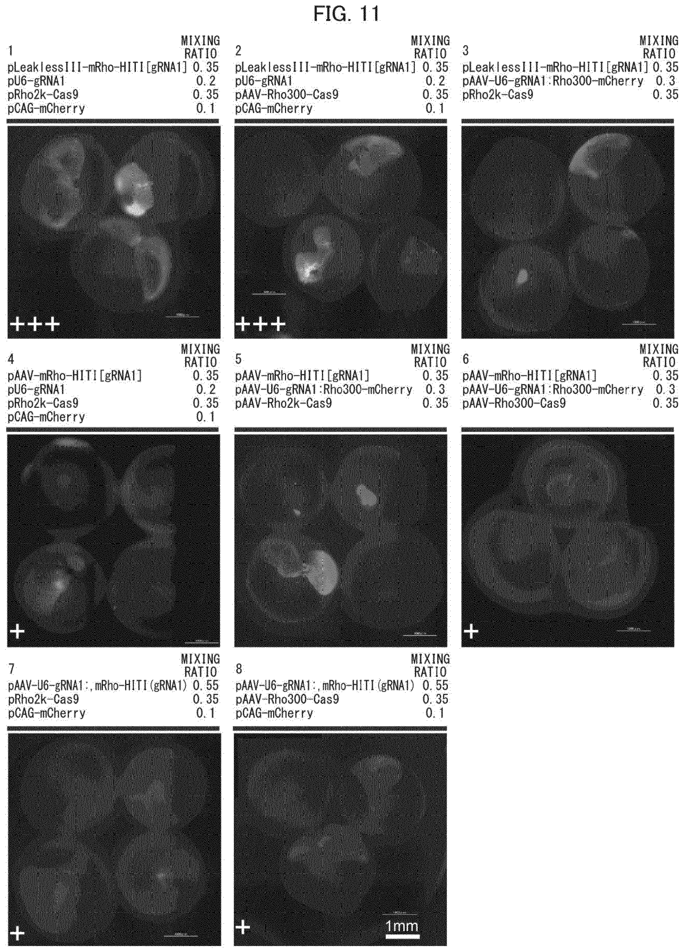

This configuration makes it possible to increase (i) the probability of insertion of the normal rhodopsin gene from the donor DNA to a genomic DNA and (ii) normal rhodopsin gene expression efficiency (for details see Examples of the present application and Fig. 11).

In an embodiment, the therapeutic agent further contains an expression vector for the gRNA and an expression vector for the designer nuclease, and these expression vectors are delivered into a cell by non-viral delivery. In this case, an expression cassette of the gRNA is placed on a vector that is different from a vector(s) on which an expression cassette of the normal rhodopsin gene and/or an expression cassette of the designer nuclease is/are placed. For example, the expression cassette of the gRNA is inserted in a plasmid that is different from a plasmid(s) in which the expression cassette of the normal rhodopsin gene and/or the expression cassette of the designer nuclease is/are inserted.

This configuration makes it possible to increase the normal rhodopsin gene expression efficiency (for details see Examples of the present application and Fig. 11). Note that such an effect is exhibited in a case where non-viral delivery is employed (electroporation etc.). In a case where viral delivery is employed, a preferred condition differs from that for non-viral delivery.

In an embodiment, the therapeutic agent further includes an expression vector for the gRNA and an expression vector for the designer nuclease, and these expression vectors are delivered into a cell by viral delivery. In this case, the donor DNA, the expression vector for the gRNA, and the expression vector for the designer nuclease are preferably each constituted by an AAV vector. Furthermore, the AAV vector is preferably at least one vector selected from the group consisting of AAV2, AAV5, AAV8, and AAV9. Such an AAV vector, which has a high affinity with respect to a photoreceptor cell, can efficiently deliver the active ingredient of the therapeutic agent to the photoreceptor cell.

In an aspect in which the AAV vector is used, the donor DNA, the expression vector for the gRNA, and the expression vector for the designer nuclease are preferably constituted by respective different AAV vectors.

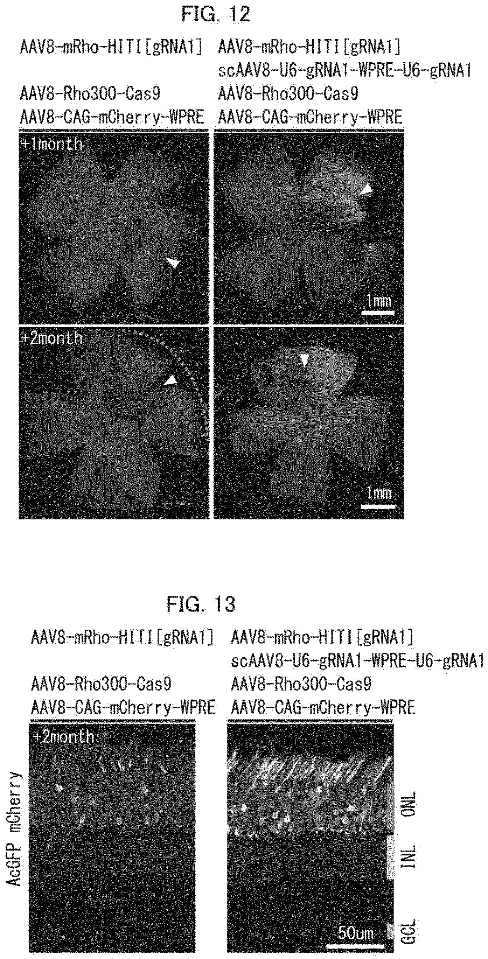

This configuration makes it possible to increase the normal rhodopsin gene expression efficiency (for details see Examples of the present application and Fig. 12).

In an embodiment, a sequence recognized by the gRNA is SEQ ID NO: 6 (gRNA1: TCTGTCTACGAAGAGCCCGTGGG) or SEQ ID NO: 8 (gRNA3: CTGAGCTCGCCAAGCAGCCTTGG). This configuration enhances efficiency of knock-in by NHEJ.

For example, in a case where the therapeutic agent in accordance with an embodiment of the present invention is applied to treatment of a human eye disease, the therapeutic agent is suitably administered by any of the following methods: subretinal injection, vitreous injection, and suprachoroidal injection. Furthermore, administration of the above therapeutic agent can bring about, to, for example, a retinitis pigmentosa patient, the following effects: (i) prevention of degeneration of a photoreceptor cell and/or recovery of a degenerated photoreceptor cell; (ii) prevention of functional degradation of a photoreceptor cell and/or recovery of a degraded photoreceptor cell function; and (iii) prevention of visual function degradation and/or recovery of a degraded visual function.

### [Advantages related to treatment of dominant rhodopsin gene mutation]

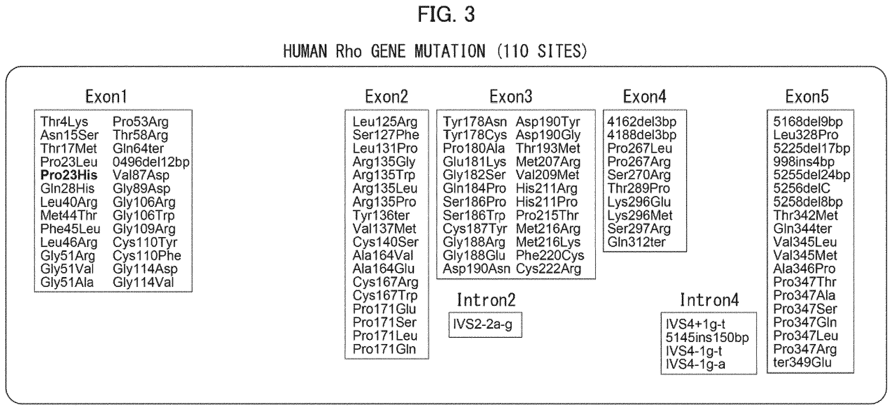

The following description will discuss, with reference to Figs. 2 and 3, advantages of application of the therapeutic agent in accordance with an embodiment of the present invention to treatment of a dominant rhodopsin gene mutation. Examples of a disease caused by a dominant rhodopsin gene mutation include retinitis pigmentosa. This disease may be a hereditary premature blindness disease caused by cell death of a photoreceptor cell (rod photoreceptor cell). Patients with retintis pigmentosa caused by a rhodopsin gene mutation account for approximately 6% of all retinitis pigmentosa patients.

Human rhodopsin protein is a photosensitive G protein-coupled receptor (GPCR) specific to a rod photoreceptor cell and composed of approximately 348 amino acids. This protein is localized in a disk membrane of a rod photoreceptor cell outer segment part. A Rhodopsin gene (Rho) encoding rhodopsin protein contains five exons and is constituted by a coding region (CDS; 10 kb), which is translated as protein, a 5' side untranslated region (1.5 kb), and a 3'-side untranslated region (a polyA addition sequence etc.; approximately 2 kb) (Fig. 2). According to a previous report, from the result of gene diagnosis of retinitis pigmentosa patients having mutations in rhodopsin genes, 110 types of gene mutations including a dominant mutation have been reported (Fig. 3).

Since ordinary genome editing technology is based on a strategy of "deleting a mutation and inserting a correct gene", it is necessary to design an appropriate gRNA and an appropriate donor DNA for each of these 110 types of gene mutations. Furthermore, genome editing technology frequently uses homologous recombination repair (HDR). HDR occurs more frequently in a dividing cell but less frequently in a non-dividing cell. In this regard, a photoreceptor cell in which a dominant rhodopsin gene mutation is expressed is a non-dividing cell. That is, it is difficult in the mainstream of ordinary genome editing technology to efficiently treat a photoreceptor cell.

In contrast, the therapeutic agent in accordance with an embodiment of the present invention knocks out a dominant rhodopsin gene mutation by knock-in of a normal rhodopsin gene between a promoter sequence and the dominant rhodopsin gene mutation. That is, the same gRNA and the same donor DNA can be used whatever gene mutation is contained in a dominant rhodopsin gene mutation (and whether the gene mutation is an unknown mutation). Furthermore, since NHEJ is used in this therapeutic agent, genome editing can be efficiently carried out also in a photoreceptor cell, which is a non-dividing cell.

### [Others]

The present invention also includes the following aspects.
[1] A therapeutic agent for a disease caused by a dominant rhodopsin gene mutation in which a dominant mutation occurs in a normal rhodopsin gene in a genome,
   the therapeutic agent comprising a donor DNA that contains a polynucleotide having the following sequences (a) to (c):
      (a) the normal rhodopsin gene;
      (b) a first reverse target DNA that is located upstream of the normal rhodopsin gene and that is cleaved by a designer nuclease; and
      (c) a second reverse target DNA that is located downstream of the normal rhodopsin gene and that is cleaved by the designer nuclease,
   where the first reverse target DNA and the second reverse target DNA each mean a sequence obtained by inverting a target sequence that is present in the genome and that is cleaved by the designer nuclease.
[2] The therapeutic agent recited in [1], wherein the donor DNA further includes the following sequence (d):
   (d) a transcriptional regulatory sequence that is located between the normal gene and the second reverse target DNA.
[3] A therapeutic agent recited in [1] or [2], further comprising at least one of the following (i) and (ii):
   (i) a gRNA or an expression vector for the gRNA; and
   (ii) the designer nuclease or an expression vector for the designer nuclease.
[4] The therapeutic agent recited in any one of [1] to [3], wherein the donor DNA further includes a sequence that causes the normal rhodopsin gene to be highly expressed.
[5] The therapeutic agent recited in any one of [1] to [4], wherein
   the donor DNA is delivered into a cell by non-viral delivery, and
   an expression cassette of the normal rhodopsin gene is inserted in a pLeaklessIII plasmid.
[6] The therapeutic agent recited in [5], wherein
   the therapeutic agent further comprises an expression vector for the gRNA and an expression vector for the designer nuclease,
   the donor DNA, the expression vector for the gRNA, and the expression vector for the designer nuclease are delivered into a cell by non-viral delivery, and
   an expression cassette of the gRNA is placed on a vector that is different from a vector(s) on which an expression cassette of the normal rhodopsin gene and/or an expression cassette of the designer nuclease is/are placed.
[7] The therapeutic agent recited in any one of [1] to [4], wherein
   the therapeutic agent further comprises an expression vector for the gRNA and an expression vector for the designer nuclease,
   the donor DNA, the expression vector for the gRNA, and the expression vector for the designer nuclease are each constituted by an AAV vector, and
   the AAV vector is preferably at least one vector selected from the group consisting of AAV2, AAV5, AAV8, and AAV9.
[8] The therapeutic agent recited in [7], wherein the donor DNA, the expression vector for the gRNA, and the expression vector for the designer nuclease are constituted by respective different AAV vectors.
[9] The therapeutic agent recited in any one of [1] to [9], wherein a sequence that is recognized by the gRNA is represented by SEQ ID NO: 6 or SEQ ID NO: 8.

<1> A method for treating a disease caused by a dominant gene mutation in which a dominant mutation occurs in a normal gene in a genome,
   the method including the step of administering a therapeutic agent to an administration subject such as a human or a non-human mammal (cattle, a pig, a sheep, a goat, a horse, a dog, a cat, a rabbit, a mouse, a rat, etc.),
   the therapeutic agent comprising a donor DNA that contains a polynucleotide having the following sequences (a) to (c):
      (a) the normal gene;
      (b) a first reverse target DNA that is located upstream of the normal gene and that is cleaved by a designer nuclease; and
      (c) a second reverse target DNA that is located downstream of the normal gene and that is cleaved by the designer nuclease,
   where the first reverse target DNA and the second reverse target DNA each mean a sequence obtained by inverting a target sequence that is present in the genome and that is cleaved by the designer nuclease.
<2> The method recited in <1>, wherein the donor DNA further includes the following sequence (d):
   (d) a transcriptional regulatory sequence that is located between the normal gene and the second reverse target DNA.
<3> The method recited in <1> or <2>, wherein the therapeutic agent further comprises at least one of the following (i) and (ii):
   (i) a gRNA or an expression vector for the gRNA; and
   (ii) the designer nuclease or an expression vector for the designer nuclease.
<4> The method recited in any one of <1> to <3>, wherein the therapeutic agent is used for a non-dividing cell.
<5> The method recited in any one of <1> to <4>, wherein the normal gene is at least one gene selected from the group consisting of a rhodopsin gene, a peripherin gene, a peripherin gene, a BEST1 gene, and an OPTN gene.
<6> The method recited in any one of <1> to <5>, wherein the disease is at least one disease selected from the group consisting of retinitis pigmentosa, macular dystrophy, and hereditary glaucoma.

(1) A therapeutic agent for a disease caused by a dominant gene mutation in which a dominant mutation occurs in a normal gene in a genome,
   the therapeutic agent comprising a donor DNA that contains a polynucleotide having the following sequences (a) to (c):
      (a) the normal gene;
      (b) a first reverse target DNA that is located upstream of the normal gene and that is cleaved by a designer nuclease; and
      (c) a second reverse target DNA that is located downstream of the normal gene and that is cleaved by the designer nuclease,
   where the first reverse target DNA and the second reverse target DNA each mean a sequence obtained by inverting a target sequence that is present in the genome and that is cleaved by the designer nuclease.
(2) The therapeutic agent recited in (1), wherein the donor DNA further includes the following sequence (d):
   (d) a transcriptional regulatory sequence that is located between the normal gene and the second reverse target DNA.
(3) A therapeutic agent recited in (1) or (2), further comprising at least one of the following (i) and (ii):
   (i) a gRNA or an expression vector for the gRNA; and
   (ii) the designer nuclease or an expression vector for the designer nuclease.
(4) The therapeutic agent recited in any one of (1) to (3), wherein the therapeutic agent is used for a non-dividing cell.
(5) The therapeutic agent recited in any one of (1) to (4), wherein the normal gene is at least one gene selected from the group consisting of a rhodopsin gene, a peripherin gene, a BEST1 gene, and an OPTN gene.
(6) The therapeutic agent recited in any one of (1) to (5), wherein the disease is at least one disease selected from the group consisting of retinitis pigmentosa, macular dystrophy, and hereditary glaucoma.

### Examples

### [Material]

### [Expression cassette]

Expression cassettes below were produced, by a usual method, as expression cassettes to be incorporated into a vector.

### 1. Expression cassette of nuclease

Cas9 derived from Streptococcus pyogenes (SpCas9) (a sequence encoding SpCas9: SEQ ID NO: 1, Uniprot Accession No. Q99ZW2) was used as a nuclease. In order that the nuclease would be specifically expressed in a rod photoreceptor cell, a region of 300 bp (SEQ ID NO: 2, gene position: chr 22: 56,231,474-56,231,769) or 2.2 kbp (SEQ ID NO: 3, gene position: chr 22: 56,231,473-56,233,726) in a bovine-derived rhodopsin promoter was used as a promoter (Gouras P et al. (1994) "Reporter gene expression in cones in transgenic mice carrying bovine rhodopsin promoter/lacZ transgenes," Visual Neuroscience, Vol. 11 (Issue 6), pp. 1227-1231; Matsuda T & Cepko CL (2007) "Controlled expression of transgenes introduced by in vivo electroporation," PNAS, Vol. 104 (No. 3), pp. 1027-1032; Onishi A et al. (2010) "The orphan nuclear hormone receptor ERRβ controls rod photoreceptor survival," PNAS, Vol. 107 (No.25), pp. 11579-11584.). Furthermore, a polyA addition sequence of rabbit β-globin (SEQ ID NO: 4, gene position: chr 1: 146,236,661-146,237,138) was used as a polyA addition sequence. The polyA addition sequence can be changed to another versatile polyA addition sequence (such as SV40pA or HGH pA).

An expression cassette in which the 300-bp region of the bovine rhodopsin promoter, the sequence encoding SpCas9, and the polyA addition sequence are arranged in order from upstream is referred to as "Rho300-Cas9". Similarly, an expression cassette in which the 2.2-kbp region of the bovine rhodopsin promoter, the sequence encoding SpCas9, and the polyA addition sequence are arranged in order from upstream is referred to as "Rho2k-Cas9".

### 2. Expression cassette of gRNA

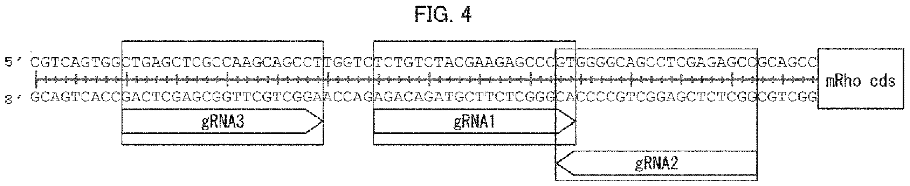

A gRNA sequence is constituted as a complex of (i) a crRNA, which is a recognition sequence of a target nucleic acid, and (ii) a tracerRNA, which activates cleavage. An SpCas9 PAM sequence (NGG) gRNA search engine was used (http:/ /crispr.technology/) to select a sequence recognized by the crRNA. A tracerRNA sequence was represented by 5'-GUUUUAGAGCUAGAAAUAGCAAGUUAAAAUAAGGCUAGUCCG UUAUCAACUUGAAAAAGUGGCACCGAGUCGGUGC-3' (SEQ ID NO: 5). A search range was within 100 bp upstream from a translation initiation site of the exon 1. This region is located upstream of the exon 1 of a rhodopsin gene and is not evolutionarily conserved. Among highly specific candidate regions presented by the search, three regions in which overlapping of base sequences did not occur were selected. The sequences are as follows (for a positional relationship among sequences recognized by respective gRNAs, see Fig. 4):
gRNA1 recognition sequence: SEQ ID NO: 6 (TCTGTCTACGAAGAGCCCGTGGG; score: high (800))
gRNA2 recognition sequence: SEQ ID NO: 7 (GGCTCTCGAGGCTGCCCCACGGG; score: high (800))
gRNA3 recognition sequence: SEQ ID NO: 8 (CTGAGCTCGCCAAGCAGCCTTGG; score: moderate (600)).

Expression cassettes in each of which a human U6 promoter sequence (gene position chr 15: 67,840,082-67,840,309), a corresponding crRNA sequence, and a tracerRNA sequence are arranged in order from upstream are referred to as "U6-gRNA1", "U6-gRNA2", and "U6-gRNA3".

### 3. Normal rhodopsin gene

A normal mouse rhodopsin protein cDNA (SEQ ID NO: 9, Accession No. NM_145383.2) was used as a normal rhodopsin gene.

Since rhodopsin protein accounts for not less than 90% of a photoreceptor cell outer segment, the normal rhodopsin gene to be knocked in is required to be expressed in a high amount. Thus, a chimeric intron sequence derived from a human β-globin gene and an immunoglobulin gene (228 bp (SEQ ID NO: 10), see Choi op cit. and Sakurai op cit.) was located upstream of the normal rhodopsin gene. This chimeric intron sequence is used in an experiment on expression of protein of a cultured cell to achieve a higher expression amount.

In downstream of the normal rhodopsin gene, a stop codon of the normal rhodopsin gene was deleted. Furthermore, a Furin sequence and a P2A sequence (78 bp; both are self-cleaving peptide sequences; SEQ ID NO: 11), an AcGFP gene (720 bp; SEQ ID NO: 12), and a rhodopsin gene 3' untranslated region (al-Ubaidi MR et al. (1990) "Mouse Opsin: Gene structure and molecular basis of multiple transcripts," Journal of Biological Chemistry, Vol. 265 (No. 33), pp. 20563-20569.) and a 100 bp sequence downstream thereof (2158 bp in total; SEQ ID NO: 13; gene position: chr 6: 115,936,720-115,938,976) were arranged in this order. The Furin sequence and the P2A sequence cause rhodopsin protein and AcGFP protein to be expressed in conjunction with each other (these sequences, which are cleaved on a C-terminal side of the rhodopsin protein and on an N-terminal side of the AcGFP protein, do not affect other protein). The AcGFP gene is a reporter for visualizing knock-in and expression of a donor gene.

Moreover, reverse target DNAs were located upstream and downstream of the resultant sequence. A reverse target DNA is a sequence (inverted sequence) obtained by inverting the upstream and the downstream of a base sequence of the gRNA (described above). An expression cassette in which a reverse target DNA corresponding to the gRNA1 is selected is referred to as "mRho-HITI-Donor [gRNA1]". An expression cassette in which a reverse target DNA corresponding to the gRNA2 is selected is referred to as "mRho-HITI-Donor [gRNA2]". An expression cassette in which a reverse target DNA corresponding to the gRNA3 is selected is referred to as "mRho-HITI-Donor [gRNA3]".

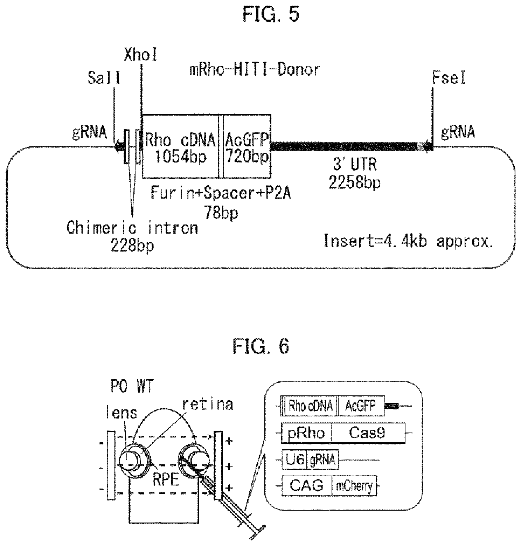

Fig. 5 schematically illustrates a structure of a donor gene containing the expression cassette (produced above) of the normal rhodopsin gene (approximately 4.4 kb). In a case where the presence of a reverse target DNA causes the normal rhodopsin gene to be inserted backwards, an inserted sequence is cleaved again by the Cas9. The cleavage and insertion is repeatedly carried out until the normal rhodopsin gene is inserted forward.

### 4. mCherry

As a reporter of whether a gene has been introduced, mCherry, which is red fluorescent protein, was used (a sequence encoding the mCherry: SEQ ID NO: 14). A CAG promoter (SEQ ID NO: 15) or a bovine-derived rhodopsin promoter 300-bp region (SEQ ID NO: 16) was used as a promoter. A polyA addition sequence of rabbit β-globin was used as a polyA addition sequence. Note that the reporter can be changed to other red fluorescent protein (mRFP, DsRed2, tdTomato, etc.). Note also that the polyA addition sequence can also be changed to another versatile polyA addition sequence (SV40pA, HGH pA, etc.).

An expression cassette in which the CAG promoter, the sequence encoding the mCherry, and the polyA addition sequence are arranged in order from upstream is referred to as "CAG-mCherry". Similarly, an expression cassette in which the 300-bp region of the bovine rhodopsin promoter, the sequence encoding the mCherry, and the polyA addition sequence are arranged in order from upstream is referred to as "Rho300-mCherry".

Note that an expression cassette of the mCherry is unnecessary for treatment based on genome editing and therefore does not need to be included in the therapeutic agent in accordance with an embodiment of the present invention.

### [Plasmid vector]

A plasmid vector having such an expression cassette as described above was produced by a usual method. The plasmid vector has a specific configuration that is described below.

### 1. Expression vector for Cas9

Out of a plasmid pCAGIG (Addgene #11159), a region extending from the CAG promoter to a polyA sequence was replaced with the Rho300-Cas9 or the Rho2k-Cas9. Thus, expression vectors "pRho300-Cas9" and "pRho2k-Cas9" were produced.

### 2. Expression vector for gRNA

The U6-gRNA1, the U6-gRNA2, or the U6-gRNA3 was inserted into a multicloning site (MCS) of a plasmid pBluescriptII. Thus, expression vectors "pU6-gRNA1", "pU6-gRNA2" and "pU6-gRNA3" were produced.

### 3. Expression vector for normal rhodopsin gene

The mRho-HITI-Donor [gRNA1], the mRho-HITI-Donor [gRNA2], or the mRho-HITI-Donor [gRNA3] was inserted into an MCS of a plasmid pLeaklessIII (Tunekawa Y et al. (2016) "Developing a de novo targeted knock-in method based on in utero electroporation into the mammalian brain," Deveopment, Vol. 143 (Issue 17), pp. 3216-3222.). Thus, expression vectors "pLeaklessIII-mRho-HITI-Donor [gRNA1]", "pLeaklessIII-mRho-HITI-Donor [gRNA2]", and "pLeaklessIII-mRho-HITI-Donor [gRNA3]" were produced.

Note here that the pLeaklessIII is a plasmid that reduces an influence of plasmid-derived endogenous transcriptional activity on an insert. In this plasmid, a CMV promoter and three SV40-polyA sequences are arranged upstream of the MCS. The plasmid-derived endogenous transcriptional activity is therefore stopped prior to an MCS sequence into which the insert is to be inserted.

### 4. Expression vector for mCherry

A GFP sequence was removed by restriction enzyme treatment from the CAG promoter of the plasmid pCAGIG, and then the CAG-mCherry or the Rho300-mCherry was inserted. Thus, expression vectors "pCAG-mCherry" and "pRho300-mCherry" were produced.

### [Example 1: Study of efficiency of knock-in by gRNA to be used]

Knock-in efficiency of the normal rhodopsin gene was studied for each of the gRNA1, the gRNA2, and the gRNA3 each described above.

Solutions containing the expression vectors (described above) were mixed at ratios shown in Table 1 so that three types of knock-in solutions were prepared.

### [Table 1]

**Table 1**

| HITI-gRNA1 | HITI-gRNA2 | HITI-gRNA3 | Mixing ratio |
|---|---|---|---|
| pLeaklessIII-mRho-HITI-Donor [gRNA1] | pLeaklessIII-mRho-HITI-Donor [gRNA2] | pLeaklessIII-mRho-HITI-Donor [gRNA3] | 0.35 |
| pU6-gRNA1 | pU6-gRNA2 | pU6-gRNA3 | 0.2 |
| pRho2k-Cas9 | pRho2k-Cas9 | pRho2k-Cas9 | 0.35 |
| pCAG-mCherry | pCAG-mCherry | pCAG-mCherry | 0.1 |

| | | | |
|---|---|---|---|
| A **total** expression vector concentration is 5 µg/µL to 7 µg/µL. | | | |

Into the retinas of mice (6 mice) at postnatal day 0 (P0), 0.3 µL to 0.4µL of HITI-gRNA1, HITI-gRNA2, and HITI-gRNA3 were injected. The heads of the mice were placed between tweezers electrodes (CUY650-7 manufactured by Nepa Gene Co., Ltd.) so that positive poles of the electrodes were located on the retina side, and an electroporator (NEPA21 manufactured by Nepa Gene Co., Ltd.) was used to apply an electrical pulse so that the expression vectors were introduced into a cell (for details see a previous report (Matsuda op cit.; Onishi op cit.; de Melo J & Blackshaw S (2011) "In vivo Electroporation of Developing Mouse Retina," Journal of Visualized Experiments, (57), e2847)). Fig. 6 schematically shows an overview of the method described above.

A rhodopsin promoter that is incorporated into the Rho2k-Cas9 starts to be activated at P7 to p10 after differentiation into a rod photoreceptor cell. That is, the Cas9 starts to be expressed in the rod photoreceptor cell, which is a non-dividing cell.

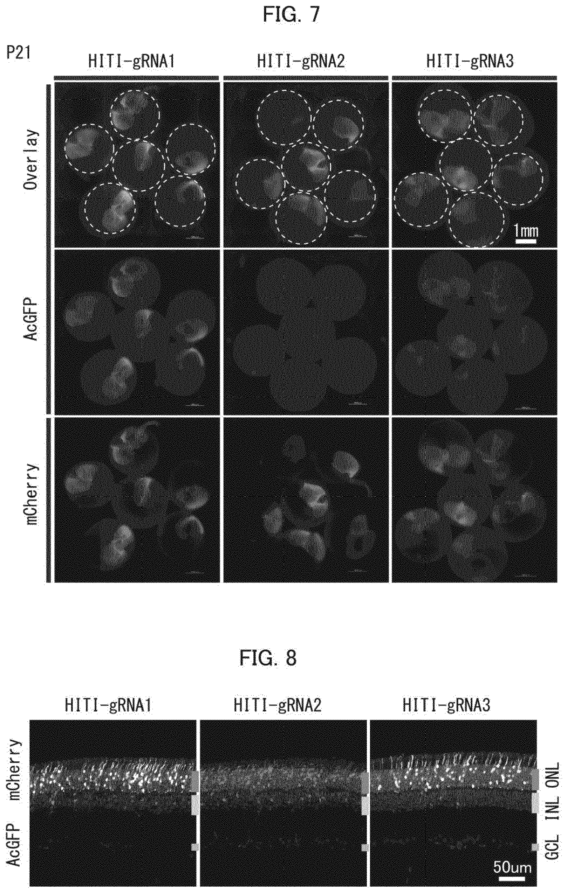

Six eyeballs were collected at P21 (cell differentiation of the retina was almost completed at that time). These eyeballs were fixed in a 4% paraformaldehyde solution (manufactured by NACALAI TESQUE, INC.) for 1 hour at room temperature while the sclera on the outside of each of the eyeballs was made in a separated (eyecup) state. Thereafter, a fluorescence stereo microscope was used to capture a fluorescent image of the GFP and the mCherry. Fig. 7 shows a result.

In a view of an overlaid image (Overlay) in the upper row of Fig. 7, a broken-line circle indicates a single eyeball. A view in the middle row of Fig. 7 shows a fluorescent image of AcGFP and illustrates a cell in which a normal rhodopsin gene has been knocked in. A view in the lower row of Fig. 7 shows a fluorescent image of the mCherry and illustrates a cell into which an expression vector has been introduced.

As illustrated in Fig. 7, green fluorescence was observed in a case where the gRNA1 and the gRNA3 were used. This result suggests that in order to increase efficiency of knock-in of the normal rhodopsin gene, it is preferable to select the gRNA1 or the gRNA3 as the gRNA.

Next, a retinal section was produced from an eyecup identical to the above eyecup, and a fluorescent image of the AcGFP and the mCherry was observed. Fig. 8 shows a result.

As illustrated in the fluorescent image of the retinal section of Fig. 8, an AcGFP-expressing cell was observed only in an outer nuclear layer (ONL) in which the rod photoreceptor cell was localized. In contrast, an mCherry-expressing cell was also observed in an inner nuclear layer (INL) in which a horizontal cell, a bipolar cell, and an amacrine cell were distributed. This result shows that knock-in of the normal rhodopsin gene specifically occurred in the rod photoreceptor cell.

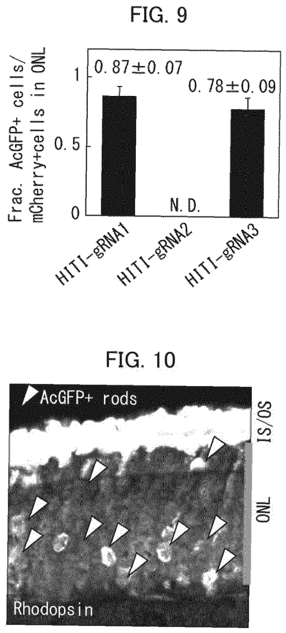

Next, a ratio between "AcGFP-expressing cells/mCherry-expressing cells" in the outer nuclear layer (ONL) was calculated from the above-obtained fluorescent image of the retinal section. Fig. 9 shows a result.

As shown in Fig. 9, it is understood that knock-in occurred with a high probability of approximately 80% to 90% in a case where the gRNA1 or the gRNA3 was used.

Next, a retinal section of a mouse into which the normal rhodopsin gene had been knocked in with use of the gRNA1 was stained with an anti-rhodopsin antibody. Fig. 10 shows a result.

As shown in Fig. 10, binding of the anti-rhodopsin antibody was observed in the AcGFP-expressing cell. That is, rhodopsin protein was observed to be expressed in a cell into which the normal rhodopsin gene had been knocked in.

### [Example 2: Study of efficiency of knock-in by configuration of expression vector]

An influence of a configuration of an expression vector on knock-in efficiency was studied. Specifically, (i) a type of plasmid into which an expression cassette is to be incorporated and (ii) a combination of expression cassettes to be incorporated into a single plasmid were changed so that an influence of the type and the combination on the knock-in efficiency was studied. For that reason, in addition to the expression vectors (described above), the following expression vectors were produced.

Note that an adeno-associated virus (AAV) vector in plasmid form and having ITR sequences at both ends (pAAV) thereof was used to produce these expression vectors. Since the pAAV has a packaging upper limit of approximately 5 kb, the expression cassettes were designed to fall within the upper limit.

### 1. pAAV-U6-gRNA1: Rho300-mCherry

The U6-gRNA1 (an expression cassette of the gRNA1) and the Rho300-mCherry (an expression cassette of the mCherry) were inserted into the pAAV. Thus, a tandem expression vector "pAAV-U6-gRNA1: Rho300-mCherry" was produced.

### 2. pAAV-U6-gRNA1: mRho-HITI-Donor [gRNA1]

The U6-gRNA1 (the expression cassette of the gRNA1) and the mRho-HITI-Donor [gRNA1](an expression cassette of the normal rhodopsin gene) were inserted into the pAAV. Thus, a tandem expression vector "pAAV-U6-gRNA1: mRho-HITI-Donor [gRNA1]" was produced.

### 3. pAAV-mRho-HITI-Donor [gRNA1]

The mRho-HITI-Donor [gRNA1](the expression cassette of the normal rhodopsin gene) was inserted into the pAAV. Thus, an expression vector "pAAV-mRho-HITI-Donor [gRNA1]" was produced.

### 4. pAAV-Rho2k-Cas9

The Rho2k-Cas9 (an expression cassette of the Cas9) was inserted into the pAAV. Thus, an expression vector "pAAV-Rho2k-Cas9" was produced.

### 5. pAAV-Rho300-Cas9

The Rho300-Cas9 was inserted into the pAAV. Thus, an expression vector "pAAV-Rho300-Cas9" was produced.

Solutions containing the expression vectors (described above) were mixed at ratios shown in Table 2 so that knock-in solutions of (1) to (8) were prepared. Note that respective features of the solutions are as follows.

(1) Control
(2) Reduction in Rho promoter from 2 kb to 300 bp
(3) Tandem between the expression cassette of the gRNA and the expression cassette of the mCherry
(4) Insertion of the expression cassette of the normal rhodopsin gene into the pAAV
(5) Combination of conditions of (3) and (4)
(6) Combination of conditions of (2) to (4)
(7) Tandem between the expression cassette of the gRNA and the expression cassette of the normal rhodopsin gene
(8) Combination of conditions of (2) and (7)

### [Table 2]

**Table 2**

| No. | Expression vector | Mixing ratio | No. | Expression vector | Mixing ratio |
|---|---|---|---|---|---|
| (1) | pLeaklessIII-mRho-HITI-Donor [gRNA1] | 0.35 | (5) | pAA V-mRho-HITI-Donor [gRNA1] | 0.35 |
| | pU6-gRNA1 | 0.2 | | pAAV-U6-gRNA1: Rho300-mCherry | 0.3 |
| | pRho2k-Cas9 | 0.35 | | pAA V-Rho2k-Cas9 | 0.35 |
| | pCAG-mCherry | 0.1 | | | |
| (2) | pLeaklessIII-mRho- | 0.35 | (6) | pAA V-mRho-HITI-Donor [gRNA1] | 0.35 |
| | HITI-Donor [gRNA1] | | | | |
| | pU6-gRNA1 | 0.2 | | pAAV-U6-gRNA1: Rho300-mCherry | 0.3 |
| | pAA V-Rho300-Cas9 | 0.35 | | pAAV-Rho300-Cas9 | 0.35 |
| | pCAG-mCherry | 0.1 | | | |
| (3) | pLeaklessIII-mRho-HITI-Donor [gRNA1] | 0.35 | (7) | pAAV-U6-gRNA1: mRho-HITI-Donor [gRNA1] | 0.55 |
| | pAAV-U6-gRNA1: Rho300-mCherry | 0.3 | | pRho2k-Cas9 | 0.35 |
| | pRho2k-Cas9 | 0.35 | | pCAG-mCherry | 0.1 |
| (4) | pAA V-mRho-HITI-Donor [gRNA1] | 0.35 | (8) | pAAV-U6-gRNA1: mRho-HITI-Donor [gRNA1] | 0.55 |
| | pU6-gRNA1 | 0.2 | | pAAV-Rho300-Cas9 | 0.35 |
| | pRho2k-Cas9 | 0.35 | | pCAG-mCherry | 0.1 |
| | pCAG-mCherry | 0.1 | | | |

| | | | | | |
|---|---|---|---|---|---|
| A total expression vector concentration is 5 µg/µL to 7 µg/µL. | | | | | |

The knock-in solutions (described above) were used to introduce the expression vectors into a cell by an electroporation method as in the case of Example 1.

At P21, an eyeball was collected. This eyeball was dissected into an eyecup, and then a fluorescence stereo microscope was used to capture a fluorescent image of the AcGFP and the mCherry. Fig. 11 shows results.

In comparison between the results of (1) and (2), there was no difference in expression intensity of the AcGFP between (a) a case where Rho2k was used as a rhodopsin promoter and (b) a case where Rho300 was used as the rhodopsin promoter. This suggests that even the rhodopsin promoter that is reduced to 300 bp does not affect the knock-in efficiency.

In comparison among the results of (1), (3), and (7), the expression intensity of the AcGFP was reduced in an expression vector in which the expression cassette of the gRNA and another cassette were combined than in an expression vector in which the expression cassette of the gRNA and another cassette were not combined. This suggests that an expression vector in which the expression cassette of the gRNA and another cassette are not combined achieves higher knock-in efficiency.

In comparison between the results of (1) and (4), the expression intensity of the AcGFP was reduced in an expression vector in which the expression cassette of the normal rhodopsin gene had been inserted into the pAAV than in an expression vector in which the expression cassette of the normal rhodopsin gene had been inserted into the pLeaklessIII. This suggests that an expression vector in which the expression cassette of the normal rhodopsin gene has been inserted into the pLeaklessIII achieves higher knock-in efficiency.

### [Example 3: Study of knock-in efficiency in viral delivery]

The knock-in efficiency in viral delivery was studied. Specifically, a type-8 AAV with which an adult rod photoreceptor cell had been observed to be infected was used to prepare AAV8 vectors so that the knock-in efficiency of these vectors was studied. For this end, the following recombinant AAV8 virus was produced.

### 1. AAV8-Rho300-Cas9

The Rho300-Cas9 (an expression cassette of the Cas9) was inserted into the pAAV. Thereafter, an AAV Helper Free Expression System was used to produce a recombinant AAV8 virus "AAV8-Rho300-Cas9".

### 2. scAAV8-U6-gRNA1-WPRE-U6-gRNA1

A tandem repeat expression cassette "U6-gRNA1-WPRE-U6-gRNA1" in which a woodchuck hepatitis virus posttranscriptional regulatory element (WPRE) was inserted was produced so that higher gRNA expression efficiency would be achieved. Furthermore, this expression cassette was inserted into a self-complementary pscAAV so that higher expression efficiency would be achieved. Thereafter, the AAV Helper Free Expression System was used to produce a recombinant scAAV8 virus "scAAV8-U6-gRNA1-WPRE-U6-gRNA1". Note that the WPRE, which is a sequence that increases stability of mRNA sent from a nucleus to a cytoplasm and promotes maturation of the mRNA, enhances (i) packaging into a virus, (ii) the virus titer, and (iii) expression of a transgene.

### 3. AAV8-mRho-HITI-Donor [gRNA1]

The mRho-HITI-Donor [gRNA1](an expression cassette of the normal rhodopsin gene) was inserted into the pAAV. Thereafter, the AAV Helper Free Expression System was used to produce a recombinant AAV8 virus "AAV8-mRho-HITI-Donor [gRNA1]".

### 4. AAV8-CAG-mCherry-WPRE

The CAG-mCherry (an expression cassette of the mCherry) and the WPRE were inserted into the pAAV. Thereafter, the AAV Helper Free Expression System was used to produce a recombinant AAV8 virus "AAV8-CAG-mCherry-WPRE".

These recombinant AAV8 viruses were used to introduce the normal rhodopsin gene into a mouse. Table 3 shows the expression vectors used for the introduction and the gene copy numbers.

### [Table 3]

**Table 3**

| Expression vector | Gene copy number [GC] | |
|---|---|---|
| | Test Example 1 | Test Example 2 |
| AAV8-mRho-HITI-Donor [gRNA1] | 7.80×10¹¹ | 7.80×10¹¹ |
| AAV8-Rho300-Cas9 | 7.80×10¹¹ | 7.80×10¹¹ |
| AAV8-CAG-mCherry-WPRE | 1.95×10¹¹ | 1.95×10¹¹ |
| scAAV8-U6-gRNA1-WPRE-U6-gRNA1 | - | 1.95×10¹¹ |

Subretinal injection of recombinant AAV8 viruses of Test Example 1 and Test Example 2 was carried out with respect to 3-month-old mice so that the mice were infected with the recombinant AAV8 viruses. The retinas were collected at 1 month and 2 months after the infection so that flat-mount specimens were produced. Thereafter, a fluorescent image of the AcGFP and the mCherry were captured as in the case of Example 2. Fig. 12 shows a result.

A view in the left column of Fig. 12 shows a fluorescent image of the retina of the mouse infected with the recombinant AAV8 virus of Test Example 1 (upper row: 1 month after infection, lower row: 2 months after infection). A view in the right column of Fig. 12 shows a fluorescent image of the retina of the mouse infected with the recombinant AAV8 virus of Test Example 2 (upper row: 1 month after infection, lower row: 2 months after infection). Arrow heads in Fig. 12 indicate locations at which subretinal injection of the recombinant AAV8 viruses was carried out.

A dotted line region in the lower row of the left column indicates a region in which a trace amount of AcGFP expression is observed. Test Example 1 is a negative control into which no expression vector for the gRNA is introduced. However, it is considered that the AcGFP derived from transcriptional activity of an AAV ITR was observed. That is, in Test Example 1, it is considered that knock-in of the normal rhodopsin gene actually does not occur.

As illustrated in Fig. 12, a range in which the AcGFP was expressed is 1/4 of the retina at 1 month after the infection and was extended to approximately 2/3 of the retina at 2 months after the infection. This is related to time taken for the mouse to be infected with an AAV virus and for the AAV virus to move into a nucleus so as to express a transgene.

Next, a section of the retina at 2 months after the infection was produced, and a fluorescent image of the AcGFP and the mCherry was observed. Fig. 13 shows a result. The left column of Fig. 13 is a view illustrating a fluorescent image of the retina of the mouse infected with the recombinant AAV8 virus of Test Example 1. The right column of Fig. 13 is a view illustrating a fluorescent image of the retina of the mouse infected with the recombinant AAV8 virus of Test Example 2.

As illustrated in Fig. 13, a cell expressing the AcGFP at a level substantially equal to that in Example 2 (Fig. 8) was observed in a retinal section at 2 months after infection with the recombinant AAV8 virus of Test Example 2. A cell expressing the AcGFP was also observed in a retinal section at 2 months after infection with the recombinant AAV8 virus of Test Example 1. However, this observed cell is considered to be derived from the transcriptional activity of the AAV ITR.

### [Example 4: Gene therapy for Rho^{P23H} retinitis pigmentosa model mouse]

A knock-in mouse with Rho^{P23H} was used as a retinitis pigmentosa model mouse, and the Rho^{P23H} is the most frequently observed in human Rho mutations (see Sakami S et al. (2011) "Probing Mechanisms of Photoreceptor Degeneration in a New Mouse Model of the Common Form of Autosomal Dominant Retinitis Pigmentosa due to P23H Opsin Mutations," Journal of Biological Chemistry, Vol. 286 (No. 12)," pp 10551-10567). In the knock-in mouse, the 23rd proline residue of the exon 1 of an Rho gene has been replaced with a histidine residue. In the knock-in mouse, retinal degeneration occurs due to endoplasmic reticulum stress that is caused because rhodopsin protein is not folded into a correct structure (see Chiang WC et al. (2015) "Robust Endoplasmic Reticulum-Associated Degradation of Rhodopsin Precedes Retinal Degeneration," Molecular Neurobiology, Vol. 52 (Issue 1); pp. 679-695.).

In a mouse (Rho^{P23H/P23H}) that has this mutation homozygously, most of rod photoreceptor cells degenerate and undergo cell death at P10 to P20. Thereafter, at the age of 1 month, a nerve cell layer (outer nuclear layer: ONL) in which a photoreceptor cell is present is thinned. Thus, the mouse is useful in detecting a rod photoreceptor cell survived by treatment.

In contrast, in a mouse (Rho^{+/P23H}) that has this mutation heterozygously, the thickness of the ONL is substantially halved by P30. However, thereafter, the retina degenerates slowly. In this case, levels of photoreceptor cell micromorphology (a photoreceptor cell outer segment length etc.) and of an electroretinogram response are approximately half those observed in a normal mouse. Thus, the mouse is useful in function evaluation with respect to a rod photoreceptor cell survived by treatment.

### [4-1. Gene therapy for Rho^{P23H/P23H} mouse]

A knock-in solution (1) used in Example 2 was used to introduce an expression vector into one of the eyes of a Rho^{P23H/P23H} mouse at P0. The expression vector was introduced by an electroporation method as in the case of Example 4. The other of the eyes was not subjected to electroporation and served as a control.

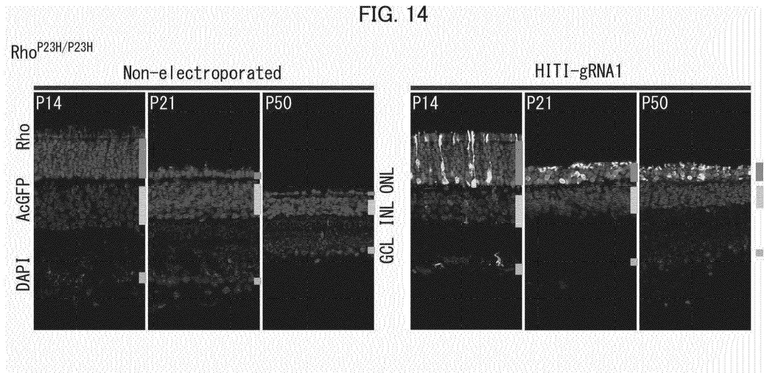

Sections of the retina were produced at time points P14, P21, and P50. An anti-rhodopsin antibody and DAPI were used to subject these sections to tissue staining. Fig. 14 shows a result.

As illustrated in Fig. 14, at P14, the ONL of the retina into which the normal rhodopsin gene had been knocked in had a thickness equal to the thickness of the ONL of the retina serving as the control. However, at P21 and P50, in the retina into which the normal rhodopsin gene had been knocked in, a cell expressing the AcGFP was present, thinning of the ONL was suppressed, and rhodopsin expression was observed in the outer segment part. In contrast, the ONL of the retina of the control was thinned. That is, knock-in of the normal rhodopsin gene suppressed degeneration of the rod photoreceptor cell in the Rho^{P23H/P23H} mouse. This suggests that (i) occurrence of knock-in of the normal rhodopsin gene and knock-out of P23H rhodopsin and (ii) expression of the normal rhodopsin resulted in avoidance of photoreceptor cell death.

### [4-2. Gene therapy for Rho^{+/P23H} mouse]

Two 1-month-old Rho^{+/P23H} mice were prepared, and the recombinant AAV8 virus used in Test Example 2 of Example 3 was injected into the left eye of each of the mice. The AAV8 virus was injected by a method similar to the method of Example 3. The AAV8 virus was not injected into the right eye of each of the mice, and the right eye served as a control.

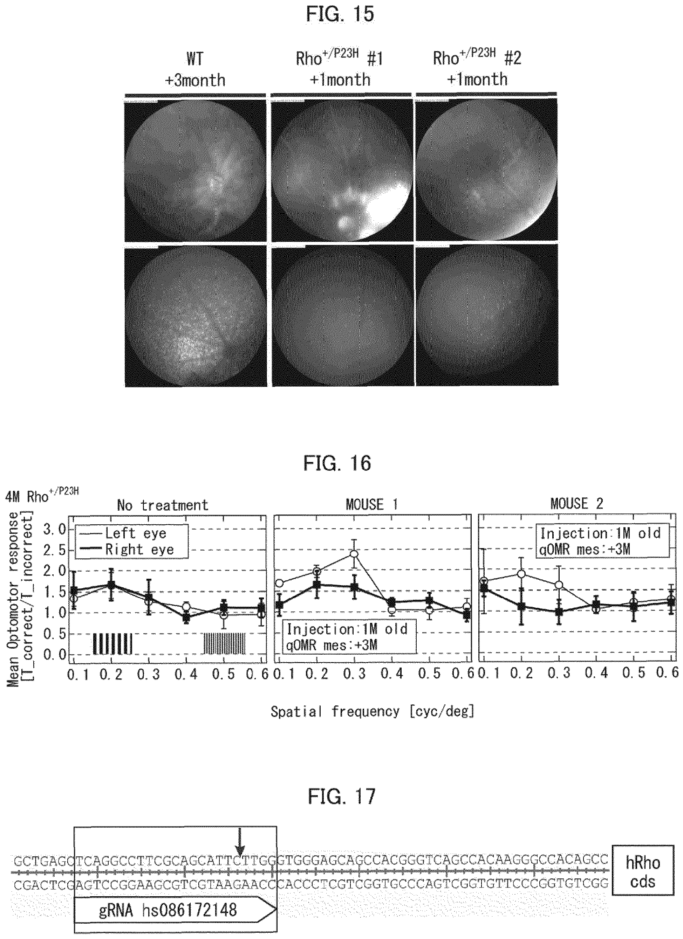

AcGFP fluorescence was observed by fluorescein fundus angiography at 1 month after infection. Fig. 15 shows a result. A view in the upper row of Fig. 15 shows a fluorescent image of the right eye, and a view in the lower row of Fig. 15 shows a fluorescent image of the left eye. A view in the left column of Fig. 15 shows a fluorescent image obtained in a case where the AAV8 virus was injected into a wild type mouse and fluorescein fundus angiography was carried out at 1 month after infection.

As illustrated in Fig. 15, though there was an individual difference between the two Rho^{+/P23H} mice, the mice each had a cell expressing the AcGFP. That is, it is suggested that normal rhodopsin is expressed instead of P23H mutated rhodopsin in these photoreceptor cells due to knock-in of the normal rhodopsin gene.

Next, at 3 months after infection, a quantitative optomotor response (qOMR) (manufactured by Phenosys) was used to carry out an optokinetic response test and quantify an optokinetic response of the right eye and the left eye. In this test, a mouse is housed in an inspection box surrounded on all sides by monitors. The monitors display striped patterns different in thickness, and the striped patterns horizontally move in a certain period of time. In a case where the mouse in the inspection box can recognize the striped patterns, the mouse moves the head in accordance with the movement of the striped patterns (the optokinetic response). The head that rotates clockwise when seen from above shows the optokinetic response derived from the left eye, and the head that rotates counterclockwise when seen from above shows the optokinetic response derived from the right eye. A camera above the head of the mouse was used to track a position of the head and quantify the optokinetic response. Fig. 16 shows results.

The left view of Fig. 16 is a graph showing a result of a test on an untreated Rho^{+/P23H} mouse into which the AAV8 virus was not injected. The central view and the right view of Fig. 16 are graphs showing results of a test on two Rho^{+/P23H} mice in each of which the AAV8 virus had been injected into the left eye.

In the wild type mouse, both the left eye and the right eye exhibited the highest response in the striped pattern of 0.2 cyc/deg, and a correct response rate (mean optomotor response) of approximately 2.0 was obtained (not illustrated). In contrast, as shown in Fig. 16, in the untreated Rho^{+/P23H} mouse (left view), both the left eye and the right eye exhibited the highest response in the striped pattern of 0.2 cyc/deg as in the case of the wild type mouse, but a correct response rate of approximately 1.5, which is lower than that of the wild type mouse, was obtained. Meanwhile, in the two Rho^{+/P23H} mice in each of which the AAV8 virus had been injected into the left eye (the central view and the right view), the left eye had a significantly high correct response rate than the right eye. These results have made it clear that knock-in of the normal rhodopsin gene suppressed functional degradation of a rod photoreceptor cell of the Rho^{+/P23H} mouse. This seems to be because (i) occurrence of knock-in of the normal rhodopsin gene and knock-out of P23H rhodopsin and (ii) expression of the normal rhodopsin gene resulted in visual function recovery, or prevented or reduced visual function degradation.

### [Example 5: Verification of gene therapy for human rhodopsin gene]

### [5-1. gRNA]

A gRNA recognition sequence that is effective in a human cell was studied so that the possibility of gene therapy for a human rhodopsin gene would be verified.

An SpCas9 gRNA search engine (http://crispr.technology/) was used to search for the gRNA recognition sequence in a region within 100 bp upstream from a translation initiation site of the exon 1 of the human rhodopsin gene. As a result, a sequence recognized by a gRNA (hs086172148) was hit (SEQ ID NO: 17: TCAGGCCTTCGCAGCATTCTTGG; score: high (800)). Fig. 17 illustrates a position of the sequence recognized by the gRNA (hs086172148). In Fig. 17, an arrow indicates a Cas9 cleavage site.

### [5-2. SSA assay]

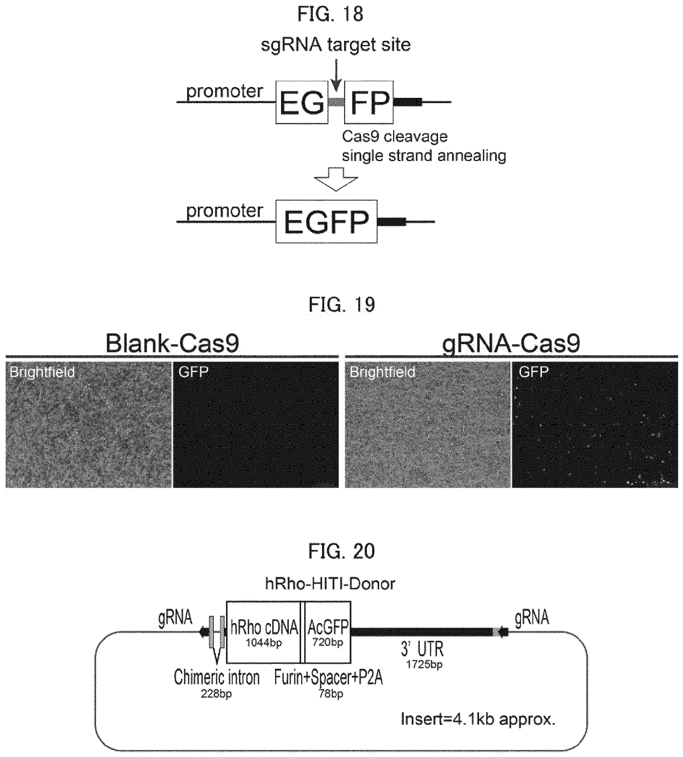

Efficiency of cleavage of the sequence recognized by the gRNA (hs086172148) was verified by a single-strand annealing (SSA) assay. Fig. 18 schematically illustrates an overview of the SSA assay.

As illustrated in Fig. 18, the gRNA (hs086172148) recognition sequence was inserted into an EGFP gene sequence of an enforced green fluorescent protein (EGFP) gene expression plasmid for the SSA assay so that a modified EGFP gene expression plasmid was produced. Base sequences in this plasmid which encode the EGFP partially overlap with each other at a position at which the base sequences are adjacent to the gRNA (hs086172148) recognition sequence. That is, the plasmid has, upstream and downstream of the gRNA (hs086172148) recognition sequence, the base sequences that partially overlap with each other.

Therefore, in a case where the gRNA (hs086172148) recognition sequence is cleaved by the Cas9, the overlapping sequences that are located at upstream and downstream of the cleavage site cause homologous recombination or single-strand annealing. This completes base sequences encoding the full-length EGFP and allows expression of the EGFP.

The produced modified EGFP gene expression plasmid, an expression vector for the gRNA (hs086172148), and an expression vector for the Cas9 were mixed so that a transfection mixed solution was prepared. This transfection mixed solution was used to transfect an HEK293 cell. The transfected HEK293 cell was cultured at 37°C and 5% CO₂ for 72 hours, and then expression of the EGFP was detected. As a control, a similar experiment was also carried out with respect to a system not containing the expression vector for the gRNA (hs086172148). Fig. 19 shows a result.

As illustrated in Fig. 19, an EGFP signal was detected in a cell transfected with both the modified EGFP gene expression plasmid and the expression vector for the gRNA (hs086172148). This shows that the gRNA (hs086172148) recognition sequence was actually cleaved by action of the gRNA (hs086172148) and the Cas9.

### [5-3. Production of donor DNA containing normal human rhodopsin gene]

The gRNA (hs086172148) recognition sequence was used to produce a donor DNA of a normal human rhodopsin gene. Fig. 20 schematically illustrates a structure of an expression cassette (approximately 4.1 kb) of the produced donor DNA.

As the normal rhodopsin gene, a sequence was used in which a stop codon had been deleted from a cDNA (1044 bp, Accession NO. NM_000539.3) of the normal human rhodopsin gene. As illustrated in Fig. 20, a chimeric intron sequence (228 bp, SEQ ID NO: 10) was located upstream of the normal rhodopsin gene. Downstream of the normal human rhodopsin gene, a Furin sequence and a P2A sequence (78 bp; SEQ ID NO: 11), an AcGFP gene (720 bp; SEQ ID NO: 12), and a 3' untranslated region of a human rhodopsin gene and a 100 bp sequence downstream thereof (1725 bp in total, SEQ ID NO: 18) were arranged in this order. Furthermore, reverse target DNAs corresponding to the gRNA (hs086172148) were located upstream and downstream of the resultant sequence so that the expression cassette of the donor DNA of the normal human rhodopsin gene was produced.

An expression vector for the normal human rhodopsin gene can be produced by inserting the expression cassette produced above into a viral or non-viral plasmid. Gene introduction efficiency of the donor DNA in a human photoreceptor cell can be specifically evaluated by introducing the produced expression vector for the normal human rhodopsin gene into, for example, the organ-cultured human retina or the human retinal organoid.

### [Example 6: Verification of gene therapy for gene which is different from rhodopsin gene and in which dominant mutation occurs]

The possibility of gene treatment for a gene which is different from the rhodopsin gene and in which a dominant mutation occurs was verified. Specifically, knock-in efficiency of a normal peripherin (Peripherin, Prph2) gene was studied in a mouse. A dominant mutation peripherin gene is a causative gene of retinitis pigmentosa and macular dystrophy, and not less than 90 types of different disease mutations have been reported so far.

### [6-1. gRNA]

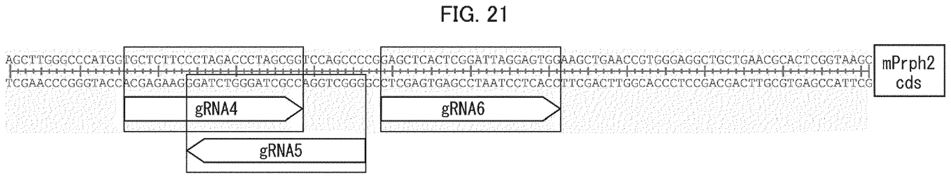

The SpCas9 gRNA search engine (http://crispr.technology/) was used to search for a gRNA recognition sequence in a region within 100 bp upstream from a translation initiation site of the exon 1 of a mouse peripherin gene, the region being not evolutionarily preserved. As a result, three gRNA recognition sequences were hit. The sequences are as follows (for a positional relationship among gRNA recognition sequences with respect to respective gRNAs, see Fig. 21):
gRNA 4 recognition sequence: SEQ ID NO: 19 (TGCTCTTCCCTAGACCCTAGCGG; score: high (800))
gRNA 5 recognition sequence: SEQ ID NO: 20 (GGGCTGGACCGCTAGGGTCTAGG; score: high (900))
gRNA 6 recognition sequence: SEQ ID NO: 21 (GAGCTCACTCGGATTAGGAGTGG; score: high (800))

For each of the gRNA4 to the gRNA6, an expression cassette was produced in which a human U6 promoter sequence, a crRNA sequence with respect to a corresponding one of the recognition sequences, and a tracerRNA sequence were arranged in order from upstream. This expression cassette was inserted into an MCS of a plasmid pBluescriptII, so that expression vectors for the gRNA4 to the gRNA6 (pU6-gRNA4, pU6-gRNA5, and pU6-gRNA6) were produced.

### [6-2. Normal peripherin gene]

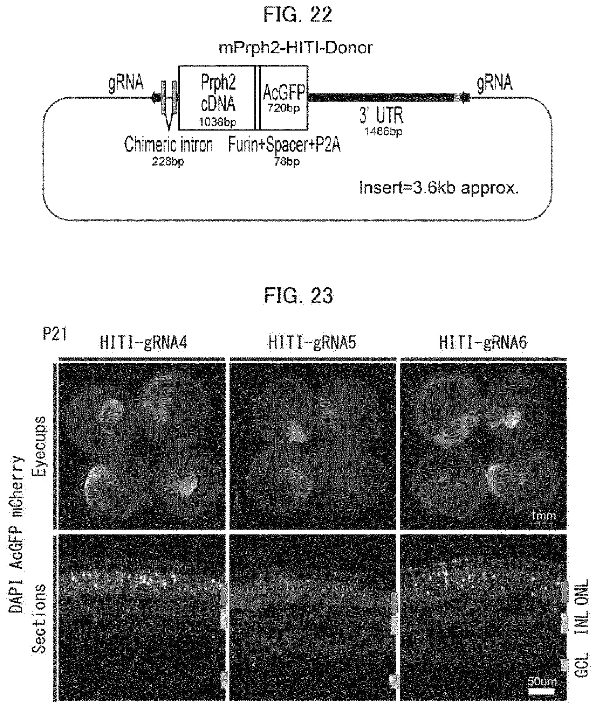

A donor DNA of a normal peripherin gene was produced as in the case of a donor DNA of a normal mouse rhodopsin gene. Fig. 22 schematically illustrates a structure of an expression cassette (approximately 3.6 kb) of the produced donor DNA.

As the normal peripherin gene, a sequence was used in which a stop codon had been deleted from a cDNA (Accession No. NM_008938.2) of normal mouse peripherin protein. A chimeric intron sequence (228 bp, SEQ ID NO: 10) was located upstream of the normal peripherin gene. Downstream of the normal peripherin gene, a Furin sequence and a P2A sequence (78 bp; SEQ ID NO: 11), an AcGFP gene (720 bp; SEQ ID NO: 12), and a 3' untranslated region of a peripherin gene and a 100 bp sequence downstream thereof (1486 bp in total, SEQ ID NO: 22, gene position: chr 17: 46,923,548-46,925,033) were arranged in this order. Furthermore, reverse target DNAs respectively corresponding to the gRNA4 to the gRNA6 were located upstream and downstream of the resultant sequence so that the expression cassette of the donor DNA of the normal peripherin gene was produced.

Expression vectors for the normal peripherin gene (pLeaklessIII-mPrph2-HITI-Donor [gRNA4], pLeaklessIII-mPrph2-HITI-Donor [gRNA5], and pLeaklessIII-mPrph2-HITI-Donor [gRNA6]) corresponding to the respective gRNA4 to gRNA6 were produced by inserting the expression cassette produced above into the MCS of the plasmid pLeaklessIII.

### [6-3. Study of knock-in efficiency]

Knock-in efficiency of the normal peripherin gene was studied as in the case of Example 1 carried out with respect to the mouse rhodopsin gene.

An expression vector for any of the gRNA4 to the gRNA6 (pU6-gRNA4, pU6-gRNA5, or pU6-gRNA6), an expression vector for the normal peripherin gene (pLeaklessIII-mPrph2-HITI-Donor [gRNA4], pLeaklessIII-mPrph2-HITI-Donor [gRNA5], or pLeaklessIII-mPrph2-HITI-Donor [gRNA6]), an expression vector for the Cas9 (pRho2k-Cas9), and an expression vector for the mCherry (pCAG-mCherry) were mixed at a ratio similar to that in Example 1 so that three types of knock-in solutions were prepared.

Into the retinas of mice (4 mice) at postnatal day 0 (P0), 0.3 pL to 0.4µL of a knock-in solution was injected so that the expression vector was introduced into a cell by an electroporation method. At P21, four eyeballs were collected and fixed in a 4% paraformaldehyde solution (manufactured by NACALAI TESQUE, INC.) for 1 hour at room temperature while the sclera on the outside of each of the eyeballs was made in a separated (eyecup) state. Thereafter, a fluorescence stereo microscope was used to capture a fluorescent image of the GFP and the mCherry. Furthermore, a retinal section was produced from an eyecup identical to the above eyecup, and a fluorescent image of the AcGFP and the mCherry was observed. Fig. 23 shows a result (upper row: eyecup, lower row: retinal section).

As illustrated in Fig. 23, green fluorescence was observed in a case where the gRNA4 was used and in a case where the gRNA6 was used. This result suggests that use of the gRNA 4 or the gRNA 6 allows the normal peripherin gene to be knocked in. Furthermore, as illustrated in the lower row of Fig. 23, an AcGFP-expressing cell was observed only in an outer nuclear layer (ONL) in which a rod photoreceptor cell was localized. In contrast, an mCherry-expressing cell was also observed in an inner nuclear layer (INL) in which a horizontal cell, a bipolar cell, and an amacrine cell were distributed. This result shows that knock-in of the normal peripherin gene specifically occurred in the rod photoreceptor cell.

### Industrial Applicability

The present invention can be used to, for example treat a disease caused by a dominant gene mutation.

### Reference Signs List

- 1:: Normal gene
- 2a:: First reverse target DNA
- 2b:: Second reverse target DNA
- 6:: Target sequence
- 7:: Dominant gene mutation
- 20:: Donor DNA
- A:: Upstream
- B:: Downstream

## Claims

1. A therapeutic agent for a disease caused by a dominant gene mutation in which a dominant mutation occurs in a normal gene in a genome,
said therapeutic agent comprising a donor DNA that contains a polynucleotide having the following sequences (a) to (c):
(a) the normal gene;
(b) a first reverse target DNA that is located upstream of the normal gene and that is cleaved by a designer nuclease; and
(c) a second reverse target DNA that is located downstream of the normal gene and that is cleaved by the designer nuclease,
where the first reverse target DNA and the second reverse target DNA each mean a sequence obtained by inverting a target sequence that is present in the genome and that is cleaved by the designer nuclease.

2. The therapeutic agent as set forth in claim 1, wherein the donor DNA further includes the following sequence (d):
(d) a transcriptional regulatory sequence that is located between the normal gene and the second reverse target DNA.

3. A therapeutic agent as set forth in claim 1 or 2, further comprising at least one of the following (i) and (ii):
(i) a gRNA or an expression vector for the gRNA; and
(ii) the designer nuclease or an expression vector for the designer nuclease.

4. The therapeutic agent as set forth in any one of claims 1 to 3, wherein the therapeutic agent is used for a non-dividing cell.

5. The therapeutic agent as set forth in any one of claims 1 to 4, wherein the normal gene is at least one gene selected from the group consisting of a rhodopsin gene, a peripherin gene, a BEST1 gene, and an OPTN gene.

6. The therapeutic agent as set forth in any one of claims 1 to 5, wherein the disease is at least one disease selected from the group consisting of retinitis pigmentosa, macular dystrophy, and hereditary glaucoma.


SEQUENCE LISTING

<110>  RIKEN

<120>  Agent for treatment of disease derived from dominant mutation
       gene

<130>  RK20180PCT

<150>  JP 2019-130199
<151>  2019-07-12

<160>  22

<170>  PatentIn version 3.5

<210>  1
<211>  4143
<212>  DNA
<213>  Streptococcus pyogenes

<400>  1
   atggacaaga agtattctat cggactggac atcgggacta atagcgtcgg gtgggccgtg      60
   atcactgacg agtacaaggt gccctctaag aagttcaagg tgctcgggaa caccgaccgg     120
   cattccatca agaaaaatct gatcggagct ctcctctttg attcagggga gaccgctgaa     180
   gcaacccgcc tcaagcggac tgctagacgg cggtacacca ggaggaagaa ccggatttgt     240
   taccttcaag agatattctc caacgaaatg gcaaaggtcg acgacagctt cttccatagg     300
   ctggaagaat cattcctcgt ggaagaggat aagaagcatg aacggcatcc catcttcggt     360
   aatatcgtcg acgaggtggc ctatcacgag aaatacccaa ccatctacca tcttcgcaaa     420
   aagctggtgg actcaaccga caaggcagac ctccggctta tctacctggc cctggcccac     480
   atgatcaagt tcagaggcca cttcctgatc gagggcgacc tcaatcctga caatagcgat     540
   gtggataaac tgttcatcca gctggtgcag acttacaacc agctctttga agagaacccc     600
   atcaatgcaa gcggagtcga tgccaaggcc attctgtcag cccggctgtc aaagagccgc     660
   agacttgaga atcttatcgc tcagctgccg ggtgaaaaga aaaatggact gttcgggaac     720
   ctgattgctc tttcacttgg gctgactccc aatttcaagt ctaatttcga cctggcagag     780
   gatgccaagc tgcaactgtc caaggacacc tatgatgacg atctcgacaa cctcctggcc     840
   cagatcggtg accaatacgc cgaccttttc cttgctgcta agaatctttc tgacgccatc     900
   ctgctgtctg acattctccg cgtgaacact gaaatcacca aggcccctct ttcagcttca     960
   atgattaagc ggtatgatga gcaccaccag gacctgaccc tgcttaaggc actcgtccgg    1020
   cagcagcttc cggagaagta caaggaaatc ttctttgacc agtcaaagaa tggatacgcc    1080
   ggctacatcg acggaggtgc ctcccaagag gaattttata agtttatcaa acctatcctt    1140
   gagaagatgg acggcaccga agagctcctc gtgaaactga atcgggagga tctgctgcgg    1200
   aagcagcgca ctttcgacaa tgggagcatt ccccaccaga tccatcttgg ggagcttcac    1260
   gccatccttc ggcgccaaga ggacttctac cctttcttta aggacaacag ggagaagatt 1320
   gagaaaattc tcactttccg catcccctac tacgtgggac ccctcgccag aggaaatagc 1380
   cggtttgctt ggatgaccag aaagtcagaa gaaactatca ctccctggaa cttcgaagag 1440
   gtggtggaca agggagccag cgctcagtca ttcatcgaac ggatgactaa cttcgataag 1500
   aacctcccca atgagaaggt cctgccgaaa cattccctgc tctacgagta ctttaccgtg 1560
   tacaacgagc tgaccaaggt gaaatatgtc accgaaggga tgaggaagcc cgcattcctg 1620
   tcaggcgaac aaaagaaggc aattgtggac cttctgttca agaccaatag aaaggtgacc 1680
   gtgaagcagc tgaaggagga ctatttcaag aaaattgaat gcttcgactc tgtggagatt 1740
   agcggggtcg aagatcggtt caacgcaagc ctgggtacct accatgatct gcttaagatc 1800
   atcaaggaca aggattttct ggacaatgag gagaacgagg acatccttga ggacattgtc 1860
   ctgactctca ctctgttcga ggaccgggaa atgatcgagg agaggcttaa gacctacgcc 1920
   catctgttcg acgataaagt gatgaagcaa cttaaacgga gaagatatac cggatgggga 1980
   cgccttagcc gcaaactcat caacggaatc cgggacaaac agagcggaaa gaccattctt 2040
   gatttcctta agagcgacgg attcgctaat cgcaacttca tgcaacttat ccatgatgat 2100
   tccctgacct ttaaggagga catccagaag gcccaagtgt ctggacaagg tgactcactg 2160
   cacgagcata tcgcaaatct ggctggttca cccgctatta agaagggtat tctccagacc 2220
   gtgaaagtcg tggacgagct ggtcaaggtg atgggtcgcc ataaaccaga gaacattgtc 2280
   atcgagatgg ccagggaaaa ccagactacc cagaagggac agaagaacag cagggagcgg 2340
   atgaaaagaa ttgaggaagg gattaaggag ctcgggtcac agatccttaa agagcacccg 2400
   gtggaaaaca cccagcttca gaatgagaag ctctatctgt actaccttca aaatggacgc 2460
   gatatgtatg tggaccaaga gcttgatatc aacaggctct cagactacga cgtggaccac 2520
   atcgtccctc agagcttcct caaagacgac tcaattgaca ataaggtgct gactcgctca 2580
   gacaagaacc ggggaaagtc agataacgtg ccctcagagg aagtcgtgaa aaagatgaag 2640
   aactattggc gccagcttct gaacgcaaag ctgatcactc agcggaagtt cgacaatctc 2700
   actaaggctg agaggggcgg actgagcgaa ctggacaaag caggattcat taaacggcaa 2760
   cttgtggaga ctcggcagat tactaaacat gtcgcccaaa tccttgactc acgcatgaat 2820
   accaagtacg acgaaaacga caaacttatc cgcgaggtga aggtgattac cctgaagtcc 2880
   aagctggtca gcgatttcag aaaggacttt caattctaca aagtgcggga gatcaataac 2940
   tatcatcatg ctcatgacgc atatctgaat gccgtggtgg gaaccgccct gatcaagaag 3000
   tacccaaagc tggaaagcga gttcgtgtac ggagactaca aggtctacga cgtgcgcaag 3060
   atgattgcca aatctgagca ggagatcgga aaggccaccg caaagtactt cttctacagc 3120
   aacatcatga atttcttcaa gaccgaaatc acccttgcaa acggtgagat ccggaagagg 3180
   ccgctcatcg agactaatgg ggagactggc gaaatcgtgt gggacaaggg cagagatttc 3240
   gctaccgtgc gcaaagtgct ttctatgcct caagtgaaca tcgtgaagaa aaccgaggtg 3300
   caaaccggag gcttttctaa ggaatcaatc ctccccaagc gcaactccga caagctcatt 3360
   gcaaggaaga aggattggga ccctaagaag tacggcggat tcgattcacc aactgtggct 3420
   tattctgtcc tggtcgtggc taaggtggaa aaaggaaagt ctaagaagct caagagcgtg 3480
   aaggaactgc tgggtatcac cattatggag cgcagctcct tcgagaagaa cccaattgac 3540
   tttctcgaag ccaaaggtta caaggaagtc aagaaggacc ttatcatcaa gctcccaaag 3600
   tatagcctgt tcgaactgga gaatgggcgg aagcggatgc tcgcctccgc tggcgaactt 3660
   cagaagggta atgagctggc tctcccctcc aagtacgtga atttcctcta ccttgcaagc 3720
   cattacgaga agctgaaggg gagccccgag gacaacgagc aaaagcaact gtttgtggag 3780
   cagcataagc attatctgga cgagatcatt gagcagattt ccgagttttc taaacgcgtc 3840
   attctcgctg atgccaacct cgataaagtc cttagcgcat acaataagca cagagacaaa 3900
   ccaattcggg agcaggctga gaatatcatc cacctgttca ccctcaccaa tcttggtgcc 3960
   cctgccgcat tcaagtactt cgacaccacc atcgaccgga aacgctatac ctccaccaaa 4020
   gaagtgctgg acgccaccct catccaccag agcatcaccg gactttacga aactcggatt 4080
   gacctctcac agctcggagg ggatgaggga gctgatccca agaaaaagcg taaggttgac 4140
   tag 4143

<210> 2
<211> 296
<212> DNA
<213> Bos taurus

<400> 2
aggcctctgc tctttcccag ggtccccagc acgccccgcc ttctccccga ccacagcctt 60

ctacctggag tcaccttggc cccacctgga agccaattaa gcccctcgtt gcagcagtga 120

ggattaatat gattaataac gcccccaatc tccgaggtgc tgattcagcc gggagcttag 180

ggaggggagg tcacttcata agggcctggg gggggagttg gagccacgag tcgtccagcc 240

ggagccccgt gtggctgagc tccggcctca gaagcatccc cgggttcgcg ccgccg 296

<210> 3
<211> 2253
<212> DNA
<213> Bos taurus

<400> 3
agcttccacg ttgtcagaag cgttagcggg taacaagcaa ggccatgagt ttgagggtcg 60

gggggacagc ctggccaggg caggctgcgg tgctctggtc ttgacccagg gagtccatcg 120

tctgtccctc tgtctggcca ccaggggccg tgttagaatc aaactcttca ccttgacctc 180
tttagcgatg gacctaatca cagggccaag caccttgagc agctgcgccc cacccacccg 240
ccacacctgc ctgccccttg ctccttcccc ccagagcact taccaccagg ctccccacac 300
aagtcctgat gctgcccgct gcccgggaca gcagggctct ggagccccag gtgggctgca 360
gcgaagggag cacgtgtctg agcagagagg tgtagacatc ccaggtggta agagggacag 420
cacaagggag cctcagcgat ggaggtctca gacactggga tgcttcctgg ccacccttgc 480
gttccccacc tcggggcctt tgcactggcc gctctctctc cttggacagc tccacccctt 540
agtgctcctt ccatcctcga gttctcagag aggccttcct gggcggctcc aggcaactgt 600
gtgtcgggag gacgtcacca tctgaacagt ccctgtggtc gtttgtttca tggtttgtgg 660
gggcccctcc ccgccccccc agaagaagga cacgtcctac aggcaggggc ttggtccttc 720
ttgtccactg tgtcccccag aactgagcgt cttgtgtaga acacagtcgg tgccagataa 780
aactcacagc tggactgtgg tcattgagga ccccgaaggc caggctgccc gcgaccccac 840
ttcactccta ggaggggtct tcacgatcct attgtagatg ggggcccttg ttcgcaccct 900
gagctcgtgc tgctgttctg ggatacgcca tcacggcttg ccgatcgcac ggcacccgag 960
gcccacggcc ctaacccagc ctgaccccag tcctgggcct cggcccgtgt gctgttccca 1020
gggcccacct cccctccata tgggagtgcc cgggaccaca cctccttgct caccgcaggg 1080
atctggtgcc cggcacaggc ttggccccgt tcacctcctg gagacgaggc cttgcttcag 1140
gagccctaag agcttctgtc ccctccccgc tttggttccc tctgccctgg tcccttcctc 1200
caccatcgct cccttcagct catccttgag ggcttttgag ccaccttcct ctccccaaga 1260
tctgcccaag ttcccctgga taaagcccct ggcactaagc cccaccccag ctgaccctag 1320
cccacgaggg cccacatccc agatgggcct cgggccttca ccgggcctct gtcttcagtc 1380
tatgctgact tccccacttg gcaacctggc aaagtgcccc cctccctcaa cacattgtcc 1440
ccctggacgt ctctgcagat aggtggcctc cccgccccgg gtccagagcg ggtcttccca 1500
cacggaagtc ctcctcagcc cataagctcc tccttcctgg agcctcgggt gccccagtgg 1560
cctgcacata aatgtcggtt tgaacaagtg agtgagtgaa ctccagttca agggaacaaa 1620
tgctttttga gctcctgggc aacgcggggc ttgtgtccgg tccccggagc gccccgctgc 1680
acttgtgaag ctcacgacct ggccggtggg cactgtcagt gtgtggtgac tggataagcc 1740
cagaggtgac ccaggtgggg aagggacagc tcaggggctg agtgagggga tgaccgaagg 1800
accagcgctc tgccctccag gagctcgtgg gccaggcggg aggaaggcaa aatttgcttg 1860
ccctgagcca gagggaagtg ggcctagagc taggggtccc aatgccaggc cagggccatc 1920
agctgagatg ccagaggggg acaggggagt tgggggcagg cctctgctct ttcccagggt 1980

ccccagcacg ccccgccttc tccccgacca cagccttcta cctggagtca ccttggcccc 2040

acctggaagc caattaagcc cctcgttgca gcagtgagga ttaatatgat taataacgcc 2100

cccaatctcc gaggtgctga ttcagccggg agcttaggga ggggaggtca cttcataagg 2160

gcctgggggg ggagttggag ccacgagtcg tccagccgga gccccgtgtg gctgagctcc 2220

ggcctcagaa gcatccccgg gttcgcgccg ccg 2253

<210> 4
<211> 478
<212> DNA
<213> Oryctolagus cuniculus

<400> 4
agaacagagc ccgaccagcc ttccgtgacc cctcgcctct cccccacctg ctggaaagct 60

ccactgtcat ctcctgtttt acacccaggg aaactaagga cacagaatag gtacccagaa 120

gcagccaggt ggtcagtccc atgtccagtg gggccacagg ccctgggttg gggtgctgaa 180

catgaagcac cctccccagc cttctccaca gcctctggtg ggcatggagc cccaagtctg 240

agggcaggaa tgcctcttgg tggccaggtt tccttccaga accagaaact ctggggtggg 300

tgggggccca gccgagctgt gtttggactc cctggcctca agagccccga caggggcctc 360

agggaaagat cccctcctcc cctctccatg tccaaaatag agttcattct caggagctgg 420

atgattttgc taagattcac aatttatttt tatttattaa aatctcttac ttctgtta 478

<210> 5
<211> 76
<212> RNA
<213> Artificial Sequence

<220>
<223> tracerRNA

<400> 5
guuuuagagc uagaaauagc aaguuaaaau aaggcuaguc cguuaucaac uugaaaaagu 60

ggcaccgagu cggugc 76

<210> 6
<211> 23
<212> DNA
<213> Artificial Sequence

<220>
<223> gRNA1

<400> 6
tctgtctacg aagagcccgt ggg 23

<210> 7
<211> 23
<212> DNA

<213>  Artificial Sequence

<220>
<223>  gRNA2

<400>  7
ggctctcgag gctgccccac ggg                                               23


<210>  8
<211>  23
<212>  DNA
<213>  Artificial Sequence

<220>
<223>  gRNA3

<400>  8
ctgagctcgc caagcagcct tgg                                               23


<210>  9
<211>  1047
<212>  DNA
<213>  Mus musculus

<400>  9
atgaacggca cagagggccc caatttttat gtgcccttct ccaacgtcac aggcgtggtg      60

cggagcccct tcgagcagcc gcagtactac ctggcggaac catggcagtt ctccatgctg     120

gcagcgtaca tgttcctgct catcgtgctg ggcttcccca tcaacttcct cacgctctac     180

gtcaccgtac agcacaagaa gctgcgcaca cccctcaact acatcctgct caacttggcc     240

gtggctgacc tcttcatggt cttcggagga ttcaccacca ccctctacac atcactccat     300

ggctacttcg tctttgggcc cacaggctgt aatctcgagg gcttctttgc cacacttgga     360

ggtgaaatcg ccctgtggtc cctggtggtc ctggccattg agcgctacgt ggtggtctgc     420

aagccgatga gcaacttccg cttcggggag aatcacgcta tcatgggtgt ggtcttcacc     480

tggatcatgg cgttggcctg tgctgctccc ccactcgttg gctggtccag gtacatccct     540

gagggcatgc aatgttcatg cgggattgac tactacacac tcaagcctga ggtcaacaac     600

gaatcctttg tcatctacat gttcgtggtc cacttcacca ttcctatgat cgtcatcttc     660

ttctgctatg ggcagctggt cttcacagtc aaggaggcgg ctgcccagca gcaggagtca     720

gccaccactc agaaggcaga gaaggaagtc acccgcatgg ttatcatcat ggtcatcttc     780

ttcctgatct gctggcttcc ctacgccagt gtggccttct acatcttcac ccaccagggc     840

tccaacttcg gccccatctt catgactctg ccagctttct ttgctaagag ctcttccatc     900

tataacccgg tcatctacat catgttgaac aagcagttcc ggaactgtat gctcaccacg     960

ctgtgctgcg gcaagaatcc actgggagat gacgacgcct ctgccaccgc ttccaagacg    1020

gagaccagcc aggtggctcc agcctaa                                        1047

<210>  10
<211>  228
<212>  DNA
<213>  Artificial Sequence

<220>
<223>  chimeric intron and exon

<400>  10
agtgactctc ttaaggtagc cttgcagaag ttggtcgtga ggcactgggc aggtaagtat      60

caaggttaca agacaggttt aaggagacca atagaaactg ggcttgtcga gacagagaag     120

actcttgcgt ttctgatagg cacctattgg tcttactgac atccactttg cctttctctc     180

cacaggtgtc cactcccagt tcaattacag ctcttaaggc tagagtac                  228


<210>  11
<211>  78
<212>  DNA
<213>  Artificial Sequence

<220>
<223>  Furin+P2A

<400>  11
cgacgcaaaa gaggaagcgg agctactaac ttcagcctgc tgaagcaggc tggagacgtg      60

gaggagaacc ctggacct                                                    78


<210>  12
<211>  720
<212>  DNA
<213>  Artificial Sequence

<220>
<223>  AcGFP

<400>  12
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtaa 720

<210> 13
<211> 2257
<212> DNA
<213> Mus musculus

<400> 13
gcctggccag agactgtggc tgaaagtagg agtctcctgt ccccacacac cccagccaca 60

gcccccacca ggagcagcac ccgttgggat gaggtcatgc aggctccctc agtgttcttt 120

tctttgtttt taatgaattc atgaaagcaa aatgaggctc cccactcaat ggggacagct 180

tgacaaaggg catccatcca ccaagaccat cctcaacctg gagtccccaa ttcccggggg 240

gccagcggga tctgtacccc tccctcagct tgtctatcag gaacatgaca agtgtcccgg 300

cttagggcta aatgtctagg acagaatgga acacatagta gctgattaat aaatgctagc 360

tggatgaagg gaggaatgag tgactgactg agtggatata tgagtgaagg gattaatgga 420

agggaacatg gatgtcctca ggtgcccaac ctggcagatc cagtcatgtc tggctggaat 480

ctataagcag ttttacatac ctgccctggt tttctctgcc cccaccccca ccccagttgg 540

atctcccaaa tccagggccc tgatagaata tggctgcttc aaagacagag agatgagggg 600

agggaggggg gagggagaga gggagggagg gagacacaga gagggaatat gtgtgatgcg 660

tgtgtatgtg tgtatgtgtg tgtgtaaaca ctttgtatat aaagagtaca gctggtagtt 720

atgttacaag taacaccgac taatataatt aattaaccat cctaatggtc tctgcttgtt 780

agtgactgct tgggaattag gcagggccca agcactcaga taaggtattt ccctcagcct 840

cagtaggctt ttgcaaatga cccaggcctt caggcctgtg cagggctaga gctggattac 900

agagataaat gacagtgaca gcaacgtgag ctgcagccct taggactgag aaagcatcga 960

gaccaggggt ctccggcaag gcctaggtcc tcccttcagt atggaaacct tgcctcatgt 1020

ctctcagcct ccttggcctg tggagatcca gcccttcctc ttggcttctg gatacatttg 1080

ctcttctaca ccagcaacca agtggcaaca gttccaggcc agtatggagt tttagaagcc 1140

atgccaatat gcccaccttc agggagcagc tgagtccttg atgccaccct tgttctgaag 1200

agttcagaaa cacagtgcaa gacatgacca ggcctcatcc ttaggatgct catggatcca 1260

gttcttagct cccttgttgg atatgctgtt ttccttggcc tttggtcttt tctttatccc 1320

agagggtttt ggctttaagg ccaacaggaa ctatggggta ccagaattga gcagcctcag 1380

tctgcatccc tcctctatag aaccacagct gggccctcag caggcccaac tctgcatggg 1440

gacagaggca ttaaaagctc agctcctaca cttggtggca gtggtggtct gttgctctca 1500

agctctttca aaatggatgg aaactgggac gcttccctga cccctggtta tgaaagacta 1560

gactgtgtgg ggacaaacag tccagagtcc cggggaatgt gatagagcag ctccatcatt 1620

tttagaaacc caatttgagg cagtatagag agatggtgac ctctataagc ctctgtatct 1680

gcaaagagga gcttagacct gcccttgagg ggattatatg agatttaagg gacttatgtg 1740

gccagcctac ttcctggcat gctgaagaca ttggcacact ctggtattct agaccttggc 1800

tcagagctgc ctttactagg atactgtcac ttagcaaaag aatgggatgg agcctcagat 1860

gtggagtgac accatcttcc aagaaggaaa gggtgccagg gtctgggatg aaagcccttt 1920

ggtgctatgt tgggcaaggg cgagtgccag caaggggtta tttgcttgct ctctccatca 1980

gtgatgaggt tccatttggt cacaagaaat tcaccccaat tgctgaaaca gaggctgact 2040

attggcttat aggcatgaaa ccccactccc ctccacttca ggctggctag attaaaagct 2100

cagacctgtg accccttgtg tctctcttct gtgttgactt cactcacagg cctaacctca 2160

gaggtgtctg tagcagtccc agggccagac acccttccag aaaggggctt tccttccctt 2220

tataatcgtg gtgacaggtc agattcttat tgggcag 2257

<210> 14
<211> 711
<212> DNA
<213> Artificial Sequence

<220>
<223> mCherry

<400> 14
atggtgagca agggcgagga ggataacatg gccatcatca aggagttcat gcgcttcaag 60

gtgcacatgg agggctccgt gaacggccac gagttcgaga tcgagggcga gggcgagggc 120

cgcccctacg agggcaccca gaccgccaag ctgaaggtga ccaagggtgg ccccctgccc 180

ttcgcctggg acatcctgtc ccctcagttc atgtacggct ccaaggccta cgtgaagcac 240

cccgccgaca tccccgacta cttgaagctg tccttccccg agggcttcaa gtgggagcgc 300

gtgatgaact tcgaggacgg cggcgtggtg accgtgaccc aggactcctc cctgcaggac 360

ggcgagttca tctacaaggt gaagctgcgc ggcaccaact tcccctccga cggccccgta 420

atgcagaaga agaccatggg ctgggaggcc tcctccgagc ggatgtaccc cgaggacggc 480

gccctgaagg gcgagatcaa gcagaggctg aagctgaagg acggcggcca ctacgacgct 540

gaggtcaaga ccacctacaa ggccaagaag cccgtgcagc tgcccggcgc ctacaacgtc 600

aacatcaagt tggacatcac ctcccacaac gaggactaca ccatcgtgga acagtacgaa 660

cgcgccgagg gccgccactc caccggcggc atggacgagc tgtacaagta a 711

<210> 15
<211> 1667
<212> DNA
<213> Artificial Sequence

<220>
<223>  CAG promoter

<400>  15
   gacattgatt attgactagt tattaatagt aatcaattac ggggtcatta gttcatagcc      60
   catatatgga gttccgcgtt acataactta cggtaaatgg cccgcctggc tgaccgccca     120
   acgacccccg cccattgacg tcaataatga cgtatgttcc catagtaacg ccaataggga     180
   ctttccattg acgtcaatgg gtggactatt tacggtaaac tgcccacttg gcagtacatc     240
   aagtgtatca tatgccaagt acgcccccta ttgacgtcaa tgacggtaaa tggcccgcct     300
   ggcattatgc ccagtacatg accttatggg actttcctac ttggcagtac atctacgtat     360
   tagtcatcgc tattaccatg ggtcgaggtg agccccacgt tctgcttcac tctccccatc     420
   tcccccccct ccccaccccc aattttgtat ttatttattt tttaattatt ttgtgcagcg     480
   atgggggcgg gggggggggg ggcgcgcgcc aggcggggcg gggcggggcg aggggcgggg     540
   cggggcgagg cggagaggtg cggcggcagc caatcagagc ggcgcgctcc gaaagtttcc     600
   ttttatggcg aggcggcggc ggcggcggcc ctataaaaag cgaagcgcgc ggcgggcggg     660
   agtcgctgcg ttgccttcgc cccgtgcccc gctccgcgcc gcctcgcgcc gcccgccccg     720
   gctctgactg accgcgttac tcccacaggt gagcgggcgg gacggccctt ctcctccggg     780
   ctgtaattag cgcttggttt aatgacggct cgtttctttt ctgtggctgc gtgaaagcct     840
   taaagggctc cgggagggcc ctttgtgcgg gggggagcgg ctcggggggt gcgtgcgtgt     900
   gtgtgtgcgt ggggagcgcc gcgtgcggcc cgcgctgccc ggcggctgtg agcgctgcgg     960
   gcgcggcgcg gggctttgtg cgctccgcgt gtgcgcgagg ggagcgcggc cgggggcggt    1020
   gccccgcggt gcgggggggc tgcgagggga acaaaggctg cgtgcggggt gtgtgcgtgg    1080
   gggggtgagc agggggtgtg ggcgcggcgg tcgggctgta acccccccct gcacccccct    1140
   ccccgagttg ctgagcacgg cccggcttcg ggtgcggggc tccgtgcggg gcgtggcgcg    1200
   gggctcgccg tgccgggcgg ggggtggcgg caggtggggg tgccgggcgg ggcggggccg    1260
   cctcgggccg gggagggctc gggggagggg cgcggcggcc ccggagcgcc ggcggctgtc    1320
   gaggcgcggc gagccgcagc cattgccttt tatggtaatc gtgcgagagg gcgcagggac    1380
   ttcctttgtc ccaaatctgg cggagccgaa atctgggagg cgccgccgca ccccctctag    1440
   cgggcgcggg cgaagcggtg cggcgccggc aggaaggaaa tgggcgggga gggccttcgt    1500
   gcgtcgccgc gccgccgtcc ccttctccat ctccagcctc ggggctgccg cagggggacg    1560
   gctgccttcg ggggggacgg ggcagggcgg ggttcggctt ctggcgtgtg accggcggct    1620
   ctagagcctc tgctaaccat gttcatgcct tcttcttttt cctacag                  1667

<210>  16
<211>  589

<212>  DNA
<213>  Artificial Sequence

<220>
<223>  WPRE

<400>  16
aatcaacctc tggattacaa aatttgtgaa agattgactg gtattcttaa ctatgttgct      60

ccttttacgc tatgtggata cgctgcttta atgcctttgt atcatgctat tgcttcccgt     120

atggctttca ttttctcctc cttgtataaa tcctggttgc tgtctcttta tgaggagttg     180

tggcccgttg tcaggcaacg tggcgtggtg tgcactgtgt ttgctgacgc aacccccact     240

ggttggggca ttgccaccac ctgtcagctc ctttccggga ctttcgcttt ccccctccct     300

attgccacgg cggaactcat cgccgcctgc cttgcccgct gctggacagg ggctcggctg     360

ttgggcactg acaattccgt ggtgttgtcg gggaaatcat cgtcctttcc ttggctgctc     420

gcctatgttg ccacctggat tctgcgcggg acgtccttct gctacgtccc ttcggccctc     480

aatccagcgg accttccttc ccgcggcctg ctgccggctc tgcggcctct tccgcgtctt     540

cgccttcgcc ctcagacgag tcggatctcc ctttgggccg cctccccgc                 589


<210>  17
<211>  23
<212>  DNA
<213>  Artificial Sequence

<220>
<223>  gRNA(hs086172148)

<400>  17
tcaggccttc gcagcattct tgg                                              23


<210>  18
<211>  1725
<212>  DNA
<213>  Homo sapiens

<400>  18
   gacctgccta ggactctgtg gccgactata ggcgtctccc atcccctaca ccttccccca      60
   gccacagcca tcccaccagg agcagcgcct gtgcagaatg aacgaagtca cataggctcc     120
   ttaatttttt ttttttttta agaaataatt aatgaggctc ctcactcacc tgggacagcc     180
   tgagaaggga catccaccaa gacctactga tctggagtcc cacgttcccc aaggccagcg     240
   ggatgtgtgc ccctcctcct cccaactcat ctttcaggaa cacgaggatt cttgctttct     300
   ggaaaagtgt cccagcttag ggataagtgt ctagcacaga atggggcaca cagtaggtgc     360
   ttaataaatg ctggatggat gcaggaagga atggaggaat gaatgggaag ggagaacata     420
   tctatcctct cagaccctcg cagcagcagc aactcatact tggctaatga tatggagcag     480
   ttgtttttcc ctccctgggc ctcactttct tctcctataa aatggaaatc ccagatccct     540
   ggtcctgccg acacgcagct actgagaaga ccaaaagagg tgtgtgtgtg tctatgtgtg 600
   tgtttcagca ctttgtaaat agcaagaagc tgtacagatt ctagttaatg ttgtgaataa 660
   catcaattaa tgtaactagt taattactat gattatcacc tcctgatagt gaacattttg 720
   agattgggca ttcagatgat ggggtttcac ccaaccttgg ggcaggtttt taaaaattag 780
   ctaggcatca aggccagacc agggctgggg gttgggctgt aggcagggac agtcacagga 840
   atgcagaatg cagtcatcag acctgaaaaa acaacactgg gggaggggga cggtgaaggc 900
   caagttccca atgagggtga gattgggcct ggggtctcac ccctagtgtg gggccccagg 960
   tcccgtgcct ccccttccca atgtggccta tggagagaca ggcctttctc tcagcctctg 1020
   gaagccacct gctcttttgc tctagcacct gggtcccagc atctagagca tggagcctct 1080
   agaagccatg ctcacccgcc cacatttaat taacagctga gtccctgatg tcatccttat 1140
   ctcgaagagc ttagaaacaa agagtgggaa attccactgg gcctaccttc cttggggatg 1200
   ttcatgggcc ccagtttcca gtttcccttg ccagacaagc ccatcttcag cagttgctag 1260
   tccattctcc attctggaga atctgctcca aaaagctggc cacatctctg aggtgtcaga 1320
   attaagctgc ctcagtaact gctccccctt ctccatataa gcaaagccag aagctctagc 1380
   tttacccagc tctgcctgga gactaaggca aattgggcca ttaaaagctc agctcctatg 1440
   ttggtattaa cggtggtggg ttttgttgct ttcacactct atccacagga tagattgaaa 1500
   ctgccagctt ccacctgatc cctgaccctg ggatggctgg attgagcaat gagcagagcc 1560
   aagcagcaca gagtcccctg ggctagaggt tggaggaggc agtcctggga atgggaaaaa 1620
   ccccaacttt ggggtcatag aggcacaggt aacccataaa actgcaaaca agctttgtca 1680
   cctctcagag cttccttatc tgcaaaaaag aatcttaaaa ctgac 1725

<210> 19
<211> 23
<212> DNA
<213> Artificial Sequence

<220>
<223> gRNA4

<400> 19
tgctcttccc tagaccctag cgg 23

<210> 20
<211> 23
<212> DNA
<213> Artificial Sequence

<220>
<223> gRNA5

<400> 20

gggctggacc gctagggtct agg                                              23

<210>  21
<211>  23
<212>  DNA
<213>  Artificial Sequence

<220>
<223>  gRNA6

<400>  21
gagctcactc ggattaggag tgg                                              23

<210>  22
<211>  1486
<212>  DNA
<213>  Mus musculus

<400>  22
   tggcctgggg cctctccccc tcctcaacac ttagtggact ccagggactg tggatacccc      60
   ctttgtccag ctgaaagtcc aaatttcccg agaaagctgg tcacctactg actctccttg     120
   atgtgggcct tgaagttcag ggtccttagg gcaggttaca aacatttgtg aaacggctgc     180
   ctccagatgt gagtgactga acgatgcagc agatggccag gactgaacgc tcacaggact     240
   ggccagtcca gggctctgtc caagtgtgag tccagatctc tcataggtga ctggccacac     300
   caagggcctc tcccctcctc agtagtgtct gcctctttta agctacaagt tctgcatccc     360
   aaccatcact tgaaacataa aagcaaggtg aaaataagaa aactctaagg gccatgttgt     420
   ttgttttcct taatgatagg gttaacaggc tggtcatcat ttgcttcatg ttacacaatg     480
   ggggagaagc aatgatattt ttttaagagt attggactcc ctcagaaggt tacctccaca     540
   tagaaattca cagatggcac aggaggtaaa aactttcctt tagtaaaatc cagtgagtgt     600
   cccatacctg ccccattccc agactcgcag tcacgtactt cagctggcat gtcccagcat     660
   cctccactca ccaagtgctg ggtgagccac tgcctgagaa ggcccccggt gagtttgctg     720
   tcctttgtgg gagagatgtg gtgatcaatt aggaatctgc cacttgggga ccaagccctg     780
   cctcctggtg gtctcagatg gccacaacag tctttgtgga ctgcactgtc caggagggca     840
   aagacccctg ggagcttctg ttctccctcc cacaaagcac tatgggaatg cttccgtatc     900
   caggaagatt ctggagatag gttatctttc cgagctagcg tgtgtggaaa gttgctgttt     960
   cttctgggaa cgtcaggcaa actctcaggg gaagcaaaaa tatgattttg gtggcacctt    1020
   gtgggatggc acccacaagg actgttgtgg ccatctgaga ccaccaggag gcagggcttg    1080
   gtccccaagg gctcagctgc tgctggcctg tgctgtgtgg ttctaactct ctcaggggga    1140
   agaccagatt agagatgctg gcacacccaa cgaactatgg ccccaatgtt ggttagtatt    1200
   ctgagtctct cagagcaaga aatggcccag atgctgagcc aggcatctgt ggcggggacc    1260
   ctcggggtcc tgccgtcacc tctcaggagg gctgagacta tggtgaaaga tactctagac 1320
   ccatttctaa ctatgctgtc aaccatcgta ttaagagata ttaaaaacta tattgtatga 1380
   aaatcactgt taagtgactg tccgtctatt caacagcaca cgtcacagga tgtcaaattc 1440
   tagccagtac ctgtttaaaa aggaaggagc caggtccagc cgtgca 1486

SEQUENCE LISTING

<110>  RIKEN

<120>  Agent for treatment of disease derived from dominant mutation
       gene

<130>  RK20180PCT

<150>  JP 2019-130199
<151>  2019-07-12

<160>  22

<170>  PatentIn version 3.5

<210>  1
<211>  4143
<212>  DNA
<213>  Streptococcus pyogenes

<400>  1
   atggacaaga agtattctat cggactggac atcgggacta atagcgtcgg gtgggccgtg      60
   atcactgacg agtacaaggt gccctctaag aagttcaagg tgctcgggaa caccgaccgg     120
   cattccatca agaaaaatct gatcggagct ctcctctttg attcagggga gaccgctgaa     180
   gcaacccgcc tcaagcggac tgctagacgg cggtacacca ggaggaagaa ccggatttgt     240
   taccttcaag agatattctc caacgaaatg gcaaaggtcg acgacagctt cttccatagg     300
   ctggaagaat cattcctcgt ggaagaggat aagaagcatg aacggcatcc catcttcggt     360
   aatatcgtcg acgaggtggc ctatcacgag aaatacccaa ccatctacca tcttcgcaaa     420
   aagctggtgg actcaaccga caaggcagac ctccggctta tctacctggc cctggcccac     480
   atgatcaagt tcagaggcca cttcctgatc gagggcgacc tcaatcctga caatagcgat     540
   gtggataaac tgttcatcca gctggtgcag acttacaacc agctctttga agagaacccc     600
   atcaatgcaa gcggagtcga tgccaaggcc attctgtcag cccggctgtc aaagagccgc     660
   agacttgaga atcttatcgc tcagctgccg ggtgaaaaga aaaatggact gttcgggaac     720
   ctgattgctc tttcacttgg gctgactccc aatttcaagt ctaatttcga cctggcagag     780
   gatgccaagc tgcaactgtc caaggacacc tatgatgacg atctcgacaa cctcctggcc     840
   cagatcggtg accaatacgc cgaccttttc cttgctgcta agaatctttc tgacgccatc     900
   ctgctgtctg acattctccg cgtgaacact gaaatcacca aggcccctct ttcagcttca     960
   atgattaagc ggtatgatga gcaccaccag gacctgaccc tgcttaaggc actcgtccgg    1020
   cagcagcttc cggagaagta caaggaaatc ttctttgacc agtcaaagaa tggatacgcc    1080
   ggctacatcg acggaggtgc ctcccaagag gaattttata agtttatcaa acctatcctt    1140
   gagaagatgg acggcaccga agagctcctc gtgaaactga atcgggagga tctgctgcgg    1200
   aagcagcgca ctttcgacaa tgggagcatt ccccaccaga tccatcttgg ggagcttcac    1260
   gccatccttc ggcgccaaga ggacttctac cctttcttta aggacaacag ggagaagatt 1320
   gagaaaattc tcactttccg catcccctac tacgtgggac ccctcgccag aggaaatagc 1380
   cggtttgctt ggatgaccag aaagtcagaa gaaactatca ctccctggaa cttcgaagag 1440
   gtggtggaca agggagccag cgctcagtca ttcatcgaac ggatgactaa cttcgataag 1500
   aacctcccca atgagaaggt cctgccgaaa cattccctgc tctacgagta ctttaccgtg 1560
   tacaacgagc tgaccaaggt gaaatatgtc accgaaggga tgaggaagcc cgcattcctg 1620
   tcaggcgaac aaaagaaggc aattgtggac cttctgttca agaccaatag aaaggtgacc 1680
   gtgaagcagc tgaaggagga ctatttcaag aaaattgaat gcttcgactc tgtggagatt 1740
   agcggggtcg aagatcggtt caacgcaagc ctgggtacct accatgatct gcttaagatc 1800
   atcaaggaca aggattttct ggacaatgag gagaacgagg acatccttga ggacattgtc 1860
   ctgactctca ctctgttcga ggaccgggaa atgatcgagg agaggcttaa gacctacgcc 1920
   catctgttcg acgataaagt gatgaagcaa cttaaacgga gaagatatac cggatgggga 1980
   cgccttagcc gcaaactcat caacggaatc cgggacaaac agagcggaaa gaccattctt 2040
   gatttcctta agagcgacgg attcgctaat cgcaacttca tgcaacttat ccatgatgat 2100
   tccctgacct ttaaggagga catccagaag gcccaagtgt ctggacaagg tgactcactg 2160
   cacgagcata tcgcaaatct ggctggttca cccgctatta agaagggtat tctccagacc 2220
   gtgaaagtcg tggacgagct ggtcaaggtg atgggtcgcc ataaaccaga gaacattgtc 2280
   atcgagatgg ccagggaaaa ccagactacc cagaagggac agaagaacag cagggagcgg 2340
   atgaaaagaa ttgaggaagg gattaaggag ctcgggtcac agatccttaa agagcacccg 2400
   gtggaaaaca cccagcttca gaatgagaag ctctatctgt actaccttca aaatggacgc 2460
   gatatgtatg tggaccaaga gcttgatatc aacaggctct cagactacga cgtggaccac 2520
   atcgtccctc agagcttcct caaagacgac tcaattgaca ataaggtgct gactcgctca 2580
   gacaagaacc ggggaaagtc agataacgtg ccctcagagg aagtcgtgaa aaagatgaag 2640
   aactattggc gccagcttct gaacgcaaag ctgatcactc agcggaagtt cgacaatctc 2700
   actaaggctg agaggggcgg actgagcgaa ctggacaaag caggattcat taaacggcaa 2760
   cttgtggaga ctcggcagat tactaaacat gtcgcccaaa tccttgactc acgcatgaat 2820
   accaagtacg acgaaaacga caaacttatc cgcgaggtga aggtgattac cctgaagtcc 2880
   aagctggtca gcgatttcag aaaggacttt caattctaca aagtgcggga gatcaataac 2940
   tatcatcatg ctcatgacgc atatctgaat gccgtggtgg gaaccgccct gatcaagaag 3000
   tacccaaagc tggaaagcga gttcgtgtac ggagactaca aggtctacga cgtgcgcaag 3060
   atgattgcca aatctgagca ggagatcgga aaggccaccg caaagtactt cttctacagc 3120
   aacatcatga atttcttcaa gaccgaaatc acccttgcaa acggtgagat ccggaagagg 3180
   ccgctcatcg agactaatgg ggagactggc gaaatcgtgt gggacaaggg cagagatttc 3240
   gctaccgtgc gcaaagtgct ttctatgcct caagtgaaca tcgtgaagaa aaccgaggtg 3300
   caaaccggag gcttttctaa ggaatcaatc ctccccaagc gcaactccga caagctcatt 3360
   gcaaggaaga aggattggga ccctaagaag tacggcggat tcgattcacc aactgtggct 3420
   tattctgtcc tggtcgtggc taaggtggaa aaaggaaagt ctaagaagct caagagcgtg 3480
   aaggaactgc tgggtatcac cattatggag cgcagctcct tcgagaagaa cccaattgac 3540
   tttctcgaag ccaaaggtta caaggaagtc aagaaggacc ttatcatcaa gctcccaaag 3600
   tatagcctgt tcgaactgga gaatgggcgg aagcggatgc tcgcctccgc tggcgaactt 3660
   cagaagggta atgagctggc tctcccctcc aagtacgtga atttcctcta ccttgcaagc 3720
   cattacgaga agctgaaggg gagccccgag gacaacgagc aaaagcaact gtttgtggag 3780
   cagcataagc attatctgga cgagatcatt gagcagattt ccgagttttc taaacgcgtc 3840
   attctcgctg atgccaacct cgataaagtc cttagcgcat acaataagca cagagacaaa 3900
   ccaattcggg agcaggctga gaatatcatc cacctgttca ccctcaccaa tcttggtgcc 3960
   cctgccgcat tcaagtactt cgacaccacc atcgaccgga aacgctatac ctccaccaaa 4020
   gaagtgctgg acgccaccct catccaccag agcatcaccg gactttacga aactcggatt 4080
   gacctctcac agctcggagg ggatgaggga gctgatccca agaaaaagcg taaggttgac 4140
   tag 4143

<210> 2
<211> 296
<212> DNA
<213> Bos taurus

<400> 2
aggcctctgc tctttcccag ggtccccagc acgccccgcc ttctccccga ccacagcctt 60

ctacctggag tcaccttggc cccacctgga agccaattaa gcccctcgtt gcagcagtga 120

ggattaatat gattaataac gcccccaatc tccgaggtgc tgattcagcc gggagcttag 180

ggaggggagg tcacttcata agggcctggg gggggagttg gagccacgag tcgtccagcc 240

ggagccccgt gtggctgagc tccggcctca gaagcatccc cgggttcgcg ccgccg 296

<210> 3
<211> 2253
<212> DNA
<213> Bos taurus

<400> 3
agcttccacg ttgtcagaag cgttagcggg taacaagcaa ggccatgagt ttgagggtcg 60

gggggacagc ctggccaggg caggctgcgg tgctctggtc ttgacccagg gagtccatcg 120

tctgtccctc tgtctggcca ccaggggccg tgttagaatc aaactcttca ccttgacctc 180
tttagcgatg gacctaatca cagggccaag caccttgagc agctgcgccc cacccacccg 240
ccacacctgc ctgccccttg ctccttcccc ccagagcact taccaccagg ctccccacac 300
aagtcctgat gctgcccgct gcccgggaca gcagggctct ggagccccag gtgggctgca 360
gcgaagggag cacgtgtctg agcagagagg tgtagacatc ccaggtggta agagggacag 420
cacaagggag cctcagcgat ggaggtctca gacactggga tgcttcctgg ccacccttgc 480
gttccccacc tcggggcctt tgcactggcc gctctctctc cttggacagc tccacccctt 540
agtgctcctt ccatcctcga gttctcagag aggccttcct gggcggctcc aggcaactgt 600
gtgtcgggag gacgtcacca tctgaacagt ccctgtggtc gtttgtttca tggtttgtgg 660
gggcccctcc ccgccccccc agaagaagga cacgtcctac aggcaggggc ttggtccttc 720
ttgtccactg tgtcccccag aactgagcgt cttgtgtaga acacagtcgg tgccagataa 780
aactcacagc tggactgtgg tcattgagga ccccgaaggc caggctgccc gcgaccccac 840
ttcactccta ggaggggtct tcacgatcct attgtagatg ggggcccttg ttcgcaccct 900
gagctcgtgc tgctgttctg ggatacgcca tcacggcttg ccgatcgcac ggcacccgag 960
gcccacggcc ctaacccagc ctgaccccag tcctgggcct cggcccgtgt gctgttccca 1020
gggcccacct cccctccata tgggagtgcc cgggaccaca cctccttgct caccgcaggg 1080
atctggtgcc cggcacaggc ttggccccgt tcacctcctg gagacgaggc cttgcttcag 1140
gagccctaag agcttctgtc ccctccccgc tttggttccc tctgccctgg tcccttcctc 1200
caccatcgct cccttcagct catccttgag ggcttttgag ccaccttcct ctccccaaga 1260
tctgcccaag ttcccctgga taaagcccct ggcactaagc cccaccccag ctgaccctag 1320
cccacgaggg cccacatccc agatgggcct cgggccttca ccgggcctct gtcttcagtc 1380
tatgctgact tccccacttg gcaacctggc aaagtgcccc cctccctcaa cacattgtcc 1440
ccctggacgt ctctgcagat aggtggcctc cccgccccgg gtccagagcg ggtcttccca 1500
cacggaagtc ctcctcagcc cataagctcc tccttcctgg agcctcgggt gccccagtgg 1560
cctgcacata aatgtcggtt tgaacaagtg agtgagtgaa ctccagttca agggaacaaa 1620
tgctttttga gctcctgggc aacgcggggc ttgtgtccgg tccccggagc gccccgctgc 1680
acttgtgaag ctcacgacct ggccggtggg cactgtcagt gtgtggtgac tggataagcc 1740
cagaggtgac ccaggtgggg aagggacagc tcaggggctg agtgagggga tgaccgaagg 1800
accagcgctc tgccctccag gagctcgtgg gccaggcggg aggaaggcaa aatttgcttg 1860
ccctgagcca gagggaagtg ggcctagagc taggggtccc aatgccaggc cagggccatc 1920
agctgagatg ccagaggggg acaggggagt tgggggcagg cctctgctct ttcccagggt 1980

ccccagcacg ccccgccttc tccccgacca cagccttcta cctggagtca ccttggcccc 2040

acctggaagc caattaagcc cctcgttgca gcagtgagga ttaatatgat taataacgcc 2100

cccaatctcc gaggtgctga ttcagccggg agcttaggga ggggaggtca cttcataagg 2160

gcctgggggg ggagttggag ccacgagtcg tccagccgga gccccgtgtg gctgagctcc 2220

ggcctcagaa gcatccccgg gttcgcgccg ccg 2253

<210> 4
<211> 478
<212> DNA
<213> Oryctolagus cuniculus

<400> 4
agaacagagc ccgaccagcc ttccgtgacc cctcgcctct cccccacctg ctggaaagct 60

ccactgtcat ctcctgtttt acacccaggg aaactaagga cacagaatag gtacccagaa 120

gcagccaggt ggtcagtccc atgtccagtg gggccacagg ccctgggttg gggtgctgaa 180

catgaagcac cctccccagc cttctccaca gcctctggtg ggcatggagc cccaagtctg 240

agggcaggaa tgcctcttgg tggccaggtt tccttccaga accagaaact ctggggtggg 300

tgggggccca gccgagctgt gtttggactc cctggcctca agagccccga caggggcctc 360

agggaaagat cccctcctcc cctctccatg tccaaaatag agttcattct caggagctgg 420

atgattttgc taagattcac aatttatttt tatttattaa aatctcttac ttctgtta 478

<210> 5
<211> 76
<212> RNA
<213> Artificial Sequence

<220>
<223> tracerRNA

<400> 5
guuuuagagc uagaaauagc aaguuaaaau aaggcuaguc cguuaucaac uugaaaaagu 60

ggcaccgagu cggugc 76

<210> 6
<211> 23
<212> DNA
<213> Artificial Sequence

<220>
<223> gRNA1

<400> 6
tctgtctacg aagagcccgt ggg 23

<210> 7
<211> 23
<212> DNA

<213>  Artificial Sequence

<220>
<223>  gRNA2

<400>  7
ggctctcgag gctgccccac ggg                                               23


<210>  8
<211>  23
<212>  DNA
<213>  Artificial Sequence

<220>
<223>  gRNA3

<400>  8
ctgagctcgc caagcagcct tgg                                               23


<210>  9
<211>  1047
<212>  DNA
<213>  Mus musculus

<400>  9
atgaacggca cagagggccc caatttttat gtgcccttct ccaacgtcac aggcgtggtg      60

cggagcccct tcgagcagcc gcagtactac ctggcggaac catggcagtt ctccatgctg     120

gcagcgtaca tgttcctgct catcgtgctg ggcttcccca tcaacttcct cacgctctac     180

gtcaccgtac agcacaagaa gctgcgcaca cccctcaact acatcctgct caacttggcc     240

gtggctgacc tcttcatggt cttcggagga ttcaccacca ccctctacac atcactccat     300

ggctacttcg tctttgggcc cacaggctgt aatctcgagg gcttctttgc cacacttgga     360

ggtgaaatcg ccctgtggtc cctggtggtc ctggccattg agcgctacgt ggtggtctgc     420

aagccgatga gcaacttccg cttcggggag aatcacgcta tcatgggtgt ggtcttcacc     480

tggatcatgg cgttggcctg tgctgctccc ccactcgttg gctggtccag gtacatccct     540

gagggcatgc aatgttcatg cgggattgac tactacacac tcaagcctga ggtcaacaac     600

gaatcctttg tcatctacat gttcgtggtc cacttcacca ttcctatgat cgtcatcttc     660

ttctgctatg ggcagctggt cttcacagtc aaggaggcgg ctgcccagca gcaggagtca     720

gccaccactc agaaggcaga gaaggaagtc acccgcatgg ttatcatcat ggtcatcttc     780

ttcctgatct gctggcttcc ctacgccagt gtggccttct acatcttcac ccaccagggc     840

tccaacttcg gccccatctt catgactctg ccagctttct ttgctaagag ctcttccatc     900

tataacccgg tcatctacat catgttgaac aagcagttcc ggaactgtat gctcaccacg     960

ctgtgctgcg gcaagaatcc actgggagat gacgacgcct ctgccaccgc ttccaagacg    1020

gagaccagcc aggtggctcc agcctaa                                        1047

<210>  10
<211>  228
<212>  DNA
<213>  Artificial Sequence

<220>
<223>  chimeric intron and exon

<400>  10
agtgactctc ttaaggtagc cttgcagaag ttggtcgtga ggcactgggc aggtaagtat      60

caaggttaca agacaggttt aaggagacca atagaaactg ggcttgtcga gacagagaag     120

actcttgcgt ttctgatagg cacctattgg tcttactgac atccactttg cctttctctc     180

cacaggtgtc cactcccagt tcaattacag ctcttaaggc tagagtac                  228


<210>  11
<211>  78
<212>  DNA
<213>  Artificial Sequence

<220>
<223>  Furin+P2A

<400>  11
cgacgcaaaa gaggaagcgg agctactaac ttcagcctgc tgaagcaggc tggagacgtg      60

gaggagaacc ctggacct                                                    78


<210>  12
<211>  720
<212>  DNA
<213>  Artificial Sequence

<220>
<223>  AcGFP

<400>  12
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtaa 720

<210> 13
<211> 2257
<212> DNA
<213> Mus musculus

<400> 13
gcctggccag agactgtggc tgaaagtagg agtctcctgt ccccacacac cccagccaca 60

gcccccacca ggagcagcac ccgttgggat gaggtcatgc aggctccctc agtgttcttt 120

tctttgtttt taatgaattc atgaaagcaa aatgaggctc cccactcaat ggggacagct 180

tgacaaaggg catccatcca ccaagaccat cctcaacctg gagtccccaa ttcccggggg 240

gccagcggga tctgtacccc tccctcagct tgtctatcag gaacatgaca agtgtcccgg 300

cttagggcta aatgtctagg acagaatgga acacatagta gctgattaat aaatgctagc 360

tggatgaagg gaggaatgag tgactgactg agtggatata tgagtgaagg gattaatgga 420

agggaacatg gatgtcctca ggtgcccaac ctggcagatc cagtcatgtc tggctggaat 480

ctataagcag ttttacatac ctgccctggt tttctctgcc cccaccccca ccccagttgg 540

atctcccaaa tccagggccc tgatagaata tggctgcttc aaagacagag agatgagggg 600

agggaggggg gagggagaga gggagggagg gagacacaga gagggaatat gtgtgatgcg 660

tgtgtatgtg tgtatgtgtg tgtgtaaaca ctttgtatat aaagagtaca gctggtagtt 720

atgttacaag taacaccgac taatataatt aattaaccat cctaatggtc tctgcttgtt 780

agtgactgct tgggaattag gcagggccca agcactcaga taaggtattt ccctcagcct 840

cagtaggctt ttgcaaatga cccaggcctt caggcctgtg cagggctaga gctggattac 900

agagataaat gacagtgaca gcaacgtgag ctgcagccct taggactgag aaagcatcga 960

gaccaggggt ctccggcaag gcctaggtcc tcccttcagt atggaaacct tgcctcatgt 1020

ctctcagcct ccttggcctg tggagatcca gcccttcctc ttggcttctg gatacatttg 1080

ctcttctaca ccagcaacca agtggcaaca gttccaggcc agtatggagt tttagaagcc 1140

atgccaatat gcccaccttc agggagcagc tgagtccttg atgccaccct tgttctgaag 1200

agttcagaaa cacagtgcaa gacatgacca ggcctcatcc ttaggatgct catggatcca 1260

gttcttagct cccttgttgg atatgctgtt ttccttggcc tttggtcttt tctttatccc 1320

agagggtttt ggctttaagg ccaacaggaa ctatggggta ccagaattga gcagcctcag 1380

tctgcatccc tcctctatag aaccacagct gggccctcag caggcccaac tctgcatggg 1440

gacagaggca ttaaaagctc agctcctaca cttggtggca gtggtggtct gttgctctca 1500

agctctttca aaatggatgg aaactgggac gcttccctga cccctggtta tgaaagacta 1560

gactgtgtgg ggacaaacag tccagagtcc cggggaatgt gatagagcag ctccatcatt 1620

tttagaaacc caatttgagg cagtatagag agatggtgac ctctataagc ctctgtatct 1680

gcaaagagga gcttagacct gcccttgagg ggattatatg agatttaagg gacttatgtg 1740

gccagcctac ttcctggcat gctgaagaca ttggcacact ctggtattct agaccttggc 1800

tcagagctgc ctttactagg atactgtcac ttagcaaaag aatgggatgg agcctcagat 1860

gtggagtgac accatcttcc aagaaggaaa gggtgccagg gtctgggatg aaagcccttt 1920

ggtgctatgt tgggcaaggg cgagtgccag caaggggtta tttgcttgct ctctccatca 1980

gtgatgaggt tccatttggt cacaagaaat tcaccccaat tgctgaaaca gaggctgact 2040

attggcttat aggcatgaaa ccccactccc ctccacttca ggctggctag attaaaagct 2100

cagacctgtg accccttgtg tctctcttct gtgttgactt cactcacagg cctaacctca 2160

gaggtgtctg tagcagtccc agggccagac acccttccag aaaggggctt tccttccctt 2220

tataatcgtg gtgacaggtc agattcttat tgggcag 2257

<210> 14
<211> 711
<212> DNA
<213> Artificial Sequence

<220>
<223> mCherry

<400> 14
atggtgagca agggcgagga ggataacatg gccatcatca aggagttcat gcgcttcaag 60

gtgcacatgg agggctccgt gaacggccac gagttcgaga tcgagggcga gggcgagggc 120

cgcccctacg agggcaccca gaccgccaag ctgaaggtga ccaagggtgg ccccctgccc 180

ttcgcctggg acatcctgtc ccctcagttc atgtacggct ccaaggccta cgtgaagcac 240

cccgccgaca tccccgacta cttgaagctg tccttccccg agggcttcaa gtgggagcgc 300

gtgatgaact tcgaggacgg cggcgtggtg accgtgaccc aggactcctc cctgcaggac 360

ggcgagttca tctacaaggt gaagctgcgc ggcaccaact tcccctccga cggccccgta 420

atgcagaaga agaccatggg ctgggaggcc tcctccgagc ggatgtaccc cgaggacggc 480

gccctgaagg gcgagatcaa gcagaggctg aagctgaagg acggcggcca ctacgacgct 540

gaggtcaaga ccacctacaa ggccaagaag cccgtgcagc tgcccggcgc ctacaacgtc 600

aacatcaagt tggacatcac ctcccacaac gaggactaca ccatcgtgga acagtacgaa 660

cgcgccgagg gccgccactc caccggcggc atggacgagc tgtacaagta a 711

<210> 15
<211> 1667
<212> DNA
<213> Artificial Sequence

<220>
<223>  CAG promoter

<400>  15
   gacattgatt attgactagt tattaatagt aatcaattac ggggtcatta gttcatagcc      60
   catatatgga gttccgcgtt acataactta cggtaaatgg cccgcctggc tgaccgccca     120
   acgacccccg cccattgacg tcaataatga cgtatgttcc catagtaacg ccaataggga     180
   ctttccattg acgtcaatgg gtggactatt tacggtaaac tgcccacttg gcagtacatc     240
   aagtgtatca tatgccaagt acgcccccta ttgacgtcaa tgacggtaaa tggcccgcct     300
   ggcattatgc ccagtacatg accttatggg actttcctac ttggcagtac atctacgtat     360
   tagtcatcgc tattaccatg ggtcgaggtg agccccacgt tctgcttcac tctccccatc     420
   tcccccccct ccccaccccc aattttgtat ttatttattt tttaattatt ttgtgcagcg     480
   atgggggcgg gggggggggg ggcgcgcgcc aggcggggcg gggcggggcg aggggcgggg     540
   cggggcgagg cggagaggtg cggcggcagc caatcagagc ggcgcgctcc gaaagtttcc     600
   ttttatggcg aggcggcggc ggcggcggcc ctataaaaag cgaagcgcgc ggcgggcggg     660
   agtcgctgcg ttgccttcgc cccgtgcccc gctccgcgcc gcctcgcgcc gcccgccccg     720
   gctctgactg accgcgttac tcccacaggt gagcgggcgg gacggccctt ctcctccggg     780
   ctgtaattag cgcttggttt aatgacggct cgtttctttt ctgtggctgc gtgaaagcct     840
   taaagggctc cgggagggcc ctttgtgcgg gggggagcgg ctcggggggt gcgtgcgtgt     900
   gtgtgtgcgt ggggagcgcc gcgtgcggcc cgcgctgccc ggcggctgtg agcgctgcgg     960
   gcgcggcgcg gggctttgtg cgctccgcgt gtgcgcgagg ggagcgcggc cgggggcggt    1020
   gccccgcggt gcgggggggc tgcgagggga acaaaggctg cgtgcggggt gtgtgcgtgg    1080
   gggggtgagc agggggtgtg ggcgcggcgg tcgggctgta acccccccct gcacccccct    1140
   ccccgagttg ctgagcacgg cccggcttcg ggtgcggggc tccgtgcggg gcgtggcgcg    1200
   gggctcgccg tgccgggcgg ggggtggcgg caggtggggg tgccgggcgg ggcggggccg    1260
   cctcgggccg gggagggctc gggggagggg cgcggcggcc ccggagcgcc ggcggctgtc    1320
   gaggcgcggc gagccgcagc cattgccttt tatggtaatc gtgcgagagg gcgcagggac    1380
   ttcctttgtc ccaaatctgg cggagccgaa atctgggagg cgccgccgca ccccctctag    1440
   cgggcgcggg cgaagcggtg cggcgccggc aggaaggaaa tgggcgggga gggccttcgt    1500
   gcgtcgccgc gccgccgtcc ccttctccat ctccagcctc ggggctgccg cagggggacg    1560
   gctgccttcg ggggggacgg ggcagggcgg ggttcggctt ctggcgtgtg accggcggct    1620
   ctagagcctc tgctaaccat gttcatgcct tcttcttttt cctacag                  1667

<210>  16
<211>  589

<212>  DNA
<213>  Artificial Sequence

<220>
<223>  WPRE

<400>  16
aatcaacctc tggattacaa aatttgtgaa agattgactg gtattcttaa ctatgttgct      60

ccttttacgc tatgtggata cgctgcttta atgcctttgt atcatgctat tgcttcccgt     120

atggctttca ttttctcctc cttgtataaa tcctggttgc tgtctcttta tgaggagttg     180

tggcccgttg tcaggcaacg tggcgtggtg tgcactgtgt ttgctgacgc aacccccact     240

ggttggggca ttgccaccac ctgtcagctc ctttccggga ctttcgcttt ccccctccct     300

attgccacgg cggaactcat cgccgcctgc cttgcccgct gctggacagg ggctcggctg     360

ttgggcactg acaattccgt ggtgttgtcg gggaaatcat cgtcctttcc ttggctgctc     420

gcctatgttg ccacctggat tctgcgcggg acgtccttct gctacgtccc ttcggccctc     480

aatccagcgg accttccttc ccgcggcctg ctgccggctc tgcggcctct tccgcgtctt     540

cgccttcgcc ctcagacgag tcggatctcc ctttgggccg cctccccgc                 589


<210>  17
<211>  23
<212>  DNA
<213>  Artificial Sequence

<220>
<223>  gRNA(hs086172148)

<400>  17
tcaggccttc gcagcattct tgg                                              23


<210>  18
<211>  1725
<212>  DNA
<213>  Homo sapiens

<400>  18
   gacctgccta ggactctgtg gccgactata ggcgtctccc atcccctaca ccttccccca      60
   gccacagcca tcccaccagg agcagcgcct gtgcagaatg aacgaagtca cataggctcc     120
   ttaatttttt ttttttttta agaaataatt aatgaggctc ctcactcacc tgggacagcc     180
   tgagaaggga catccaccaa gacctactga tctggagtcc cacgttcccc aaggccagcg     240
   ggatgtgtgc ccctcctcct cccaactcat ctttcaggaa cacgaggatt cttgctttct     300
   ggaaaagtgt cccagcttag ggataagtgt ctagcacaga atggggcaca cagtaggtgc     360
   ttaataaatg ctggatggat gcaggaagga atggaggaat gaatgggaag ggagaacata     420
   tctatcctct cagaccctcg cagcagcagc aactcatact tggctaatga tatggagcag     480
   ttgtttttcc ctccctgggc ctcactttct tctcctataa aatggaaatc ccagatccct     540
   ggtcctgccg acacgcagct actgagaaga ccaaaagagg tgtgtgtgtg tctatgtgtg 600
   tgtttcagca ctttgtaaat agcaagaagc tgtacagatt ctagttaatg ttgtgaataa 660
   catcaattaa tgtaactagt taattactat gattatcacc tcctgatagt gaacattttg 720
   agattgggca ttcagatgat ggggtttcac ccaaccttgg ggcaggtttt taaaaattag 780
   ctaggcatca aggccagacc agggctgggg gttgggctgt aggcagggac agtcacagga 840
   atgcagaatg cagtcatcag acctgaaaaa acaacactgg gggaggggga cggtgaaggc 900
   caagttccca atgagggtga gattgggcct ggggtctcac ccctagtgtg gggccccagg 960
   tcccgtgcct ccccttccca atgtggccta tggagagaca ggcctttctc tcagcctctg 1020
   gaagccacct gctcttttgc tctagcacct gggtcccagc atctagagca tggagcctct 1080
   agaagccatg ctcacccgcc cacatttaat taacagctga gtccctgatg tcatccttat 1140
   ctcgaagagc ttagaaacaa agagtgggaa attccactgg gcctaccttc cttggggatg 1200
   ttcatgggcc ccagtttcca gtttcccttg ccagacaagc ccatcttcag cagttgctag 1260
   tccattctcc attctggaga atctgctcca aaaagctggc cacatctctg aggtgtcaga 1320
   attaagctgc ctcagtaact gctccccctt ctccatataa gcaaagccag aagctctagc 1380
   tttacccagc tctgcctgga gactaaggca aattgggcca ttaaaagctc agctcctatg 1440
   ttggtattaa cggtggtggg ttttgttgct ttcacactct atccacagga tagattgaaa 1500
   ctgccagctt ccacctgatc cctgaccctg ggatggctgg attgagcaat gagcagagcc 1560
   aagcagcaca gagtcccctg ggctagaggt tggaggaggc agtcctggga atgggaaaaa 1620
   ccccaacttt ggggtcatag aggcacaggt aacccataaa actgcaaaca agctttgtca 1680
   cctctcagag cttccttatc tgcaaaaaag aatcttaaaa ctgac 1725

<210> 19
<211> 23
<212> DNA
<213> Artificial Sequence

<220>
<223> gRNA4

<400> 19
tgctcttccc tagaccctag cgg 23

<210> 20
<211> 23
<212> DNA
<213> Artificial Sequence

<220>
<223> gRNA5

<400> 20

gggctggacc gctagggtct agg                                              23

<210>  21
<211>  23
<212>  DNA
<213>  Artificial Sequence

<220>
<223>  gRNA6

<400>  21
gagctcactc ggattaggag tgg                                              23

<210>  22
<211>  1486
<212>  DNA
<213>  Mus musculus

<400>  22
   tggcctgggg cctctccccc tcctcaacac ttagtggact ccagggactg tggatacccc      60
   ctttgtccag ctgaaagtcc aaatttcccg agaaagctgg tcacctactg actctccttg     120
   atgtgggcct tgaagttcag ggtccttagg gcaggttaca aacatttgtg aaacggctgc     180
   ctccagatgt gagtgactga acgatgcagc agatggccag gactgaacgc tcacaggact     240
   ggccagtcca gggctctgtc caagtgtgag tccagatctc tcataggtga ctggccacac     300
   caagggcctc tcccctcctc agtagtgtct gcctctttta agctacaagt tctgcatccc     360
   aaccatcact tgaaacataa aagcaaggtg aaaataagaa aactctaagg gccatgttgt     420
   ttgttttcct taatgatagg gttaacaggc tggtcatcat ttgcttcatg ttacacaatg     480
   ggggagaagc aatgatattt ttttaagagt attggactcc ctcagaaggt tacctccaca     540
   tagaaattca cagatggcac aggaggtaaa aactttcctt tagtaaaatc cagtgagtgt     600
   cccatacctg ccccattccc agactcgcag tcacgtactt cagctggcat gtcccagcat     660
   cctccactca ccaagtgctg ggtgagccac tgcctgagaa ggcccccggt gagtttgctg     720
   tcctttgtgg gagagatgtg gtgatcaatt aggaatctgc cacttgggga ccaagccctg     780
   cctcctggtg gtctcagatg gccacaacag tctttgtgga ctgcactgtc caggagggca     840
   aagacccctg ggagcttctg ttctccctcc cacaaagcac tatgggaatg cttccgtatc     900
   caggaagatt ctggagatag gttatctttc cgagctagcg tgtgtggaaa gttgctgttt     960
   cttctgggaa cgtcaggcaa actctcaggg gaagcaaaaa tatgattttg gtggcacctt    1020
   gtgggatggc acccacaagg actgttgtgg ccatctgaga ccaccaggag gcagggcttg    1080
   gtccccaagg gctcagctgc tgctggcctg tgctgtgtgg ttctaactct ctcaggggga    1140
   agaccagatt agagatgctg gcacacccaa cgaactatgg ccccaatgtt ggttagtatt    1200
   ctgagtctct cagagcaaga aatggcccag atgctgagcc aggcatctgt ggcggggacc    1260
   ctcggggtcc tgccgtcacc tctcaggagg gctgagacta tggtgaaaga tactctagac 1320
   ccatttctaa ctatgctgtc aaccatcgta ttaagagata ttaaaaacta tattgtatga 1380
   aaatcactgt taagtgactg tccgtctatt caacagcaca cgtcacagga tgtcaaattc 1440
   tagccagtac ctgtttaaaa aggaaggagc caggtccagc cgtgca 1486